(19) Europäisches Patentamt
European Patent Office
Office européen des brevets

(11) Numéro de publication: **0 059 146**
**B1**

(12) # FASCICULE DE BREVET EUROPEEN

(45) Date de publication du fascicule du brevet: **25.09.85**

(21) Numéro de dépôt: **82400285.1**

(22) Date de dépôt: **18.02.82**

(51) Int. Cl.⁴: **A 61 K 31/12**, A 61 K 31/215, A 61 K 31/44, C 07 C 49/737, C 07 C 49/753, C 07 C 49/513, C 07 C 49/517, C 07 C 49/743, C 07 C 69/145, C 07 C 69/24, C 07 C 45/00

(54) **Nouveaux médicaments dérivés de la 3-hydroxy dodécahydro benz (e) inden-7-one, produits dérivés de la 3-hydroxy dodécahydro benz (e) inden-7-one et leur procédé de préparation.**

(30) Priorité: **23.02.81 FR 8103520**

(43) Date de publication de la demande: **01.09.82 Bulletin 82/35**

(45) Mention de la délivrance du brevet: **25.09.85 Bulletin 85/39**

(84) Etats contractants désignés: **AT BE CH DE FR GB IT LI LU NL SE**

(56) Documents cités:
**EP - A - 0 008 951**
**CH - A - 558 343**
**FR - A - 1 359 675**
**US - A - 3 984 473**
**US - A - 3 984 474**

(73) Titulaire: **ROUSSEL-UCLAF, 35, boulevard des Invalides, F-75007 Paris (FR)**

(72) Inventeur: **Bouton, Marie-Madeleine, 47-49, Avenue du Docteur Arnold Netter, F-75012 Paris (FR)**
Inventeur: **Jacques, Jean, 9, rue Soufflot, F-75005 Paris (FR)**
Inventeur: **Pierdet, André, 10, rue Charles Baudelaire, F-93130 Noisy le Sec (FR)**

(74) Mandataire: **Bourgouin, André et al, ROUSSEL-UCLAF 111, route de Noisy Boîte postale no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne des médicaments nouveaux dérivés de la 3-hydroxy dodécahydrobenz (e) inden-7-one et les compositions les renfermant, des produits dérivés de cette 3-hydroxy dodécahydrobenz (e) inden-7-one et leur procédé de préparation.

On connaissait déjà des produits possédant le type de structure tricyclique des produits de la présente demande.

Cependant la plupart des produits déjà décrits étaient utilisés comme produits chimiques intermédiaires de synthèse.

Parmi les documents de l'art antérieur, on peut citer le brevet français BF 1 359 675, le brevet belge 663 197, le brevet suisse CH 558 343, le brevet allemand DT 1 811 693, le brevet européen 0 008 951, les brevets américains USP 3 984 473, 3 956 316 et les publications J. Org. Chem. 1969, 34 (1) p. 107; J. Chem. Soc. 1949 p 1855; J. Chem. Soc. 1962 p. 1312; J. Org. Chem. 1969, 34 (5) p. 1457; Ann. 1963 p. 153; Helv. Chim. Acta 1971, 54 (7) p. 2121.

Le brevet américain USP 3 984 474 décrit des produits tricycliques différents des produits de la présente demande et qui de plus sont présentés comme possédant une activité anti-inflammatoire, alors que les produits de la présente demande ont une activité anti-androgène.

L'invention a pour objet, à titre de médicaments et notamment de médicaments anti-androgènes, les produits de formule générale (I):

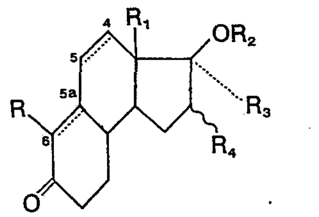

(I)

dans laquelle R représente un radical alkyle linéaire ou ramifié, ayant de 1 à 4 atomes de carbone, $R_1$ représente un radical méthyle ou éthyle, $R_2$ représente un atome d'hydrogène, un radical alkyle, alkényle ou alkynyle, linéaire ou ramifié, ayant au plus 8 atomes de carbone, éventuellement interrompu par un hétéro atome, ou un radical formyle ou acyle ayant de 2 à 18 atomes de carbone, choisi parmi les restes d'un acide aliphatique ou cycloaliphatique saturé ou insaturé, les restes cycloalkylalcanoïques, les radicaux benzoyle ou phénylalcanoyle, les radicaux acyles linéaires comportant un hétéro atome, les radicaux 3-pyridinylcarbonyle, 4-pyridinylcarbonyle, thiazolylcarbonyle, 4,5-dihydrothiazolylcarbonyle, oxazolylcarbonyle ou imidazolylcarbonyle, $R_3$ représente un atome d'hydrogène ou un radical alkyle, alkényle ou alkynyle ayant au plus 4 atomes de carbone, $R_4$ représente un atome d'hydrogène ou un radical alkyle ayant de 1 à 4 atomes de carbone, les traits pointillés indiquent la présence éventuelle d'une seconde liaison entre les carbones 4(5) et 5a(6), le trait ondulé indique que le substituant $R_4$ peut se trouver dans l'une ou l'autre des deux positions possibles α ou β. La formule (I) comprend, en mêmt temps que les produits de la série naturelle, les produits racémiques et les produits de la série antipodale.

Parmi les valeurs de R on peut citer les radicaux méthyle, éthyle, propyle, isopropyle, butyle, butyle secondaire, isobutyle, tert-butyle.

Parmi les valeurs de $R_2$ on peut citer les radicaux méthyle, éthyle, propyle, isopropyle, butyle, butyle secondaire, isobutyle, tert-butyle, pentyle, sec-pentyle, iso-pentyle, ou les radicaux hexyle, heptyle ou octyle linéaires ou ramifiés.

On peut également citer les radicaux vinyle, allyle, butényle ainsi que les radicaux éthynyle, propynyle ou butynyle.

Lorsque $R_2$ représente un radical alkyle, alkényle ou alkynyle éventuellement interrompu par un hétéro atome les valeurs préférées de ce substituant peuvent être représentées par la formule:

$$-CH_2-A-(CH_2)_n-CH_3$$

dans laquelle A représente un hétéroatome choisi parmi le soufre et l'oxygène et n est un entier choisi de 0 à 4.

La valeur préférée pour ce type de substituant est la valeur méthoxyméthyle ou un alkoxyméthyle inférieur tel que éthoxyméthyle. On peut également citer la valeur méthylthiométhyle.

Parmi les valeurs du radical acyle que peut représenter $R_2$, on peut citer les restes d'un acide aliphatique ou cycloaliphatique saturé ou insaturé tels que les radicaux acétyle, propionyle, butyryle, isobutyryle, valéryle, hexanoyle, pivaloyle, undécanoyle, acriloyle ou crotonoyle; les radicaux cyclopropyl, cyclobutyl ou cyclopentylcarbonyle, les radicaux provenant d'un reste cycloalkylalcanoïque comme les radicaux cyclopentyl ou cyclohexylacétyle ou propionyle, les radicaux benzoyle; phénylalcanoyles tels que les radicaux phénylacétyle ou phénylpropionyle. Le radical acyle peut également comporter un ou plusieurs hétéroatome choisi parmi l'azote, le soufre et l'oxygène. On peut citer par exemple les radicaux 3-pyridinyl-4-pyridin 1-thiazolyl-4,5-dihydrothiazolyl-oxazolyl- ou imidazolylcarbonyle.

Il peut également s'agir d'un radical acyle linéaire comportant un hétéroatome, tel que le radical méthoxyacétyle.

Parmi les valeurs des radicaux $R_3$ et $R_4$ on peut citer respectivement les radicaux alkyle, alkényle ou alkynyle ou simplement alkyle mentionnés cidessus.

L'invention a plus particulièrement pour objet, à titre de médicaments, les produits de formule (I) telle que décrite ci-dessus, dans laquelle R représente un radical méthyle ou éthyle et $R_3$ et $R_4$ représentent chacun un atome d'hydrogène, ainsi que ceux dans laquelle le trait pointillé en position 4(5) n'indique pas la présence d'une seconde liaison et le trait pointillé en position 5a(6) indique la présence d'une seconde liaison et ceux dans laquelle $R_2$ représente un atome d'hydrogène ou un radical méthoxyméthyle, allyle, acétyle ou butyryle.

L'invention a tout particulièrement pour objet, à titre de médicaments, les produits décrits ci-après et notamment:

– la 3a β méthyl 3β-hydroxy 6-éthyl 1,2,3,3a,4,5, 8,9,9a,9b décahydro 7-H benz (e) inden-7-one;
– la 3a β 6-diéthyl 3β-hydroxy 1,2,3,3a,4,5,8,9,9a, 9b décahydro 7-H benz (e) inden-7-one;
– la 3a β-méthyl 3β-acétoxy 6-éthyl 1,2,3,3a,4,5,8, 9,9a,9b décahydro 7H-benz (e) inden-7-one;
– la 3β-(pyridin-3-yl) carbonyloxy 6-éthyl 3a β-méthyl 1,2,3,3a,4,5,8,9,9a,9b-décahydro 7H-benz (e) inden-7-one.

L'invention a de plus pour objet, les compositions pharmaceutiques qui renferment les produits de formule générale (I) et plus particulièrement les produits préférés mentionés plus haut, à titre de principe actif.

Les produits de formule (I) de l'invention manifestent des propriétés pharmacologiques intéressantes. Ils sont doués, notamment d'une activité anti-androgène remarquable, plus particulièrement lorsqu'ils sont administrés par voie locale.

Les médicaments selon l'invention inhibent les effets des androgènes au niveau des organes périphériques sans porter atteinte au fonctionnement normal de l'hypophyse. Ils peuvent dont être utilisés chez les adolescents sans craindre un arrêt de croissance et chez les adultes sans avoir à redouter certains effets d'une castration chimique.

Ils trouvent notamment leur emploi pour le traitement des affections locales liées à une hyperandrogénocité, telles que l'acné, l'hirsutisme, la séborrhée, l'hyperpilosité.

Ils peuvent être administrés par les voies habituelles mais sont plus particulièrement destinés à l'administration par voie locale et pour cela se présentent notamment sous forme de solution, émulsions, crèmes, pommades et lotions. Alternativement, les compositions peuvent être administrées par voie orale ou rectale.

Les formes pharmaceutiques sont préparées selon les méthodes usuelles. Le principe actif peut être incorporé à des excipients habituellement employés dans ces compositions, tels que les véhicules aqueux ou non, le lactose, l'amidon, les corps gras d'origine animale ou végétale, les dérivés paraffiniques, les glycols, les divers agents mouillants, dispersants ou émulsifiants, les conservateurs.

Lorsque l'administration a lieu par les voies orale ou ractale on utilise de préférence des gélules, des comprimés ou des suppositoires.

La dose usuelle, variable selon le sujet à traiter et l'affection en cause peut être par exemple de 1 à 5 applications par jour d'une pommade contenant de 1 à 20% et préférentiellement de 2 à 10% de principe actif.

La présente demande a également pour objet à titre de produits industriels nouveaux, les produits de formule générale I':

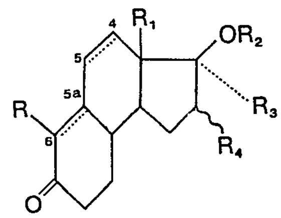

(I')

dans laquelle R, $R_1$, $R_2$, $R_3$, $R_4$, les traits pointillés et ondulés ont les significations indiquées ci-dessus, à l'exclusion des produits dans lesquels $R_4$ représente un atome d'hydrogène, le trait pointillé en position 4 (5) n'indique pas la présence d'une seconde liaison, le trait pointillé en position 5a(6) indique la présence d'une seconde liaison et

a) R représente un radical méthyle et $R_3$ représente un atome d'hydrogène et
   α) $R_2$ représente un atome d'hydrogène, un radical acétyle, benzoyle ou tert-butyle et $R_1$ représente un radical méthyle,
   β) $R_2$ représente un atome d'hydrogène ou un radical tert-butyle et $R_1$ représente un radical éthyle;
b) R représente un radical éthyle ou propyle, $R_3$ représente un atome d'hydrogène, $R_2$ représente un atome d'hydrogène ou un radical benzoyle et $R_1$ représente un radical méthyle;
c) R représente un radical butyle, $R_3$ représente un atome d'hydrogène, $R_2$ représente un atome d'hydrogène et $R_1$ représente un radical méthyle;
d) R représente un radical méthyle, $R_1$ représente un radical méthyle, $R_2$ représente un atome d'hydrogène et $R_3$ représente un radical ethyle ou méthyle;

à l'exclusion également des produits dans lesquels $R_4$ représente un atome d'hydrogène et les trait pointillés n'indiquent pas la présence de secondes liaisons et

a) R et $R_1$ représentent chacun un radical méthyle, $R_3$ représente un atome d'hydrogène et $R_2$ représente un atome d'hydrogène ou un radical acétyle ou benzoyle;
b) R et $R_1$ représentent chacun un radical méthyle, $R_2$ représente un atome d'hydrogène et $R_3$ représente un radical éthyle, propargyle ou isobutényle;
c) $R_3$ représente un atome d'hydrogène et
   α) R et $R_1$ représentent chacun un radical méthyle et $R_2$ représente un radical méthoxyméthyle;
   β) R représente un radical éthyle, $R_1$ représente un radical méthyle et $R_2$ représente un atome d'hydrogène;
   γ) R représente un radical méthyle, $R_1$ représente un radical éthyle et $R_2$ représente un atome d'hydrogène;
d) R et $R_1$ représentent chacun un radical méthyle, $R_2$ représente un atome d'hydrogène ou un radical acétyle et $R_3$ représente un radical méthyle.

Les valeurs des différents substituants R, $R_1$, $R_2$, $R_3$ et $R_4$ peuvent être définies plus spécialement comme indiqué ci-dessus.

La présente demande a plus particulièrement pour objet les produits de formule générale (I'), dans laquelle $R_4$ représente un radical alkyle ayant de 1 à 4 atomes de carbone, ainsi que les produits de formule générale (I'), dans laquelle les traits pointillés indiquent la présence de deux secondes liaisons en position 4(5) et 5a(6) et ceux dans laquelle le trait pointillé en position 5a(6) indique la présence d'une seconde liaison et les radicaux R et $R_1$ représentent chacun un radical éthyle.

La présente invention a tout particulièrement pour objet les produits décrits ci-après dans les exemples et spécialement:

– la 3a β 6-diéthyl 3β-hydroxy 1,2,3,3a,4,5,8,9,9a, 9b décahydro 7-H benz (e) inden-7-one;

– la 3a β-méthyl 3β-acétoxy 6-éthyl 1,2,3,3a,4,5,8, 9,9a,9b décahydro 7H-benz (e) inden-7-one;

– la 3β-(pyridin-3-yl) carbonyloxy 6-éthyl 3aβ-méthyl 1,2,3,3a,4,5,8,9,9a,9b-décahydro 7H-benz (e) inden-7-one.

L'invention a de plus pour objet un procédé de préparation des produits de formule (I'), telle que définie ci-dessus, caractérisé en ce que:

1) Pour préparer les produits de formule ($I'_A$)

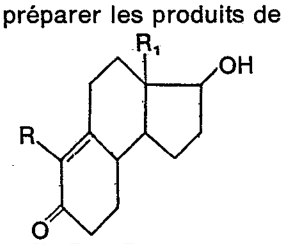

($I'_A$)

dans laquelle R et $R_1$ ont la signification indiquée ci-dessus, étant entendu que R ne peut pas représenter un radical méthyle, éthyle, n-propyle et n-butyle, lorsque $R_1$ représente un radical méthyle et que R ne peut pas représenter un radical méthyle, lorsque $R_1$ représente un radical éthyle, on fait agir un produit de formule ($II_A$)

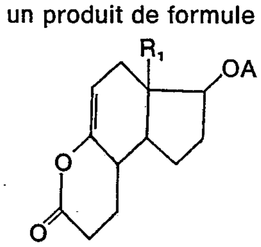

($II_A$)

avec un produit de formule ($III_A$)

$$R\text{–}CH_2\,Mg\,X \qquad (III_A)$$

formules dans lesquelles R et $R_1$ ont les significations précédentes, A représente un radical acyle ayant de 2 à 18 atomes de carbone et X représente un atome d'halogène, et saponifie si désiré le produit obtenu;

2) Pour préparer les produits de formule ($I'_B$)

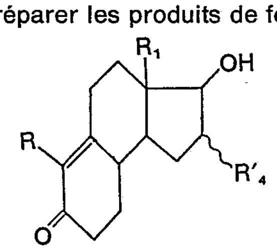

($I'_B$)

dans laquelle R et $R_1$ ont la signification indiquée ci-dessus et $R'_4$ représente un radical alkyle ayant de 1 à 4 atomes de carbone, on fait agir un produit de formule ($II_B$):

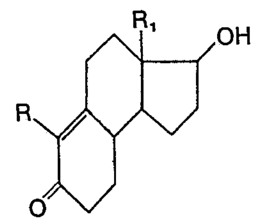

($II_B$)

dans laquelle R et $R_1$ ont les significations précédentes avec un réactif de protection du groupement cétonique pour obtenir un produit de formule ($III_B$):

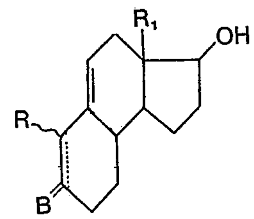

($III_B$)

dans laquelle B représente, soit un groupement cétal, soit un groupement énamine, soit un éther d'énol, le trait pointillé représente une seconde liaison lorsque B est un groupement énamine ou un éther d'énol, produit que l'on traite par un agent d'oxydation pour obtenir un produit de formule ($IV_B$):

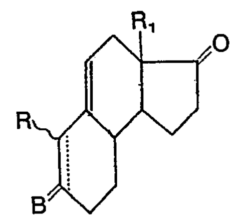

($IV_B$)

que l'on traite, en présence d'une base forte, par un produit de formule $R'_4$ I pour obtenir un produit de formule ($V_B$)

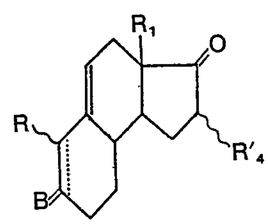

($V_B$)

que l'on soumet à l'action d'un réducteur pour obtenir un produit de formule ($V'_B$):

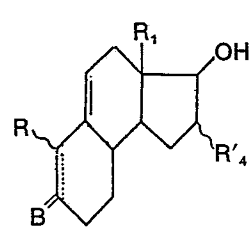

($V'_B$)

que l'on traite de manière à éliminer le radical protecteur de la fonction cétonique pour obtenir le produit de formule ($I'_B$) cherché;

3) Pour préparer les produits de formule ($I'_C$)

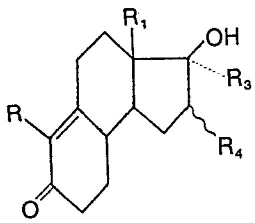

(I'C)

dans laquelle R, $R_1$, $R_3$ et $R_4$ ont la signification indiquée ci-dessus, étant entendu que lorsque $R_4$ représente un atome d'hydrogène, R et $R_1$ ne peuvent pas représenter chacun un radical méthyle lorsque $R_3$ représente un radical éthyle, on fait agir un dérivé de formule $R_3$–D dans laquelle D représente un groupement Mg–Hal, dans lequel Hal représente un atome d'halogène, ou un atome de lithium, sur un dérivé de formule ($II_C$)

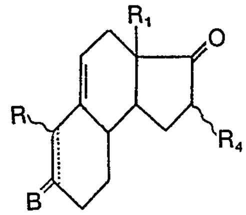

($II_C$)

dans laquelle B, R, $R_1$, $R_4$ et le trait pointillé ont la signification précédente et traite le produit obtenu de manière à éliminer le radical protecteur de la fonction cétonique;

4) Pour préparer les produits de formule ($I'_D$)

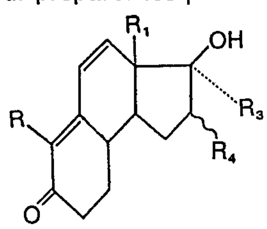

($I'_D$)

dans laquelle R, $R_1$, $R_3$ et $R_4$ ont la signification indiquée ci-dessus, on fait agir un dérivé réactif d'un groupement protecteur du groupement hydroxyle sur un produit de formule ($II_D$):

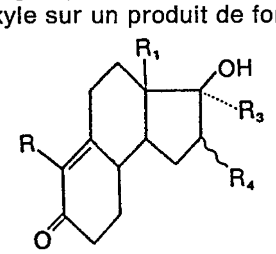

($II_D$)

dans laquelle R, $R_1$, $R_3$ et $R_4$ ont la signification indiquée ci-dessus, pour obtenir un produit de formule ($III_D$)

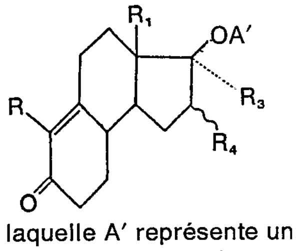

($III_D$)

dans laquelle A' représente un radical protecteur du groupement hydroxyle, que l'on traite par un réactif de bromuration pour obtenir un produit de formule ($IV_D$):

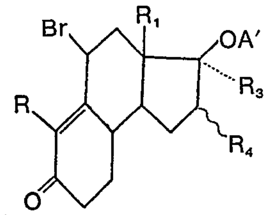

($IV_D$)

que l'on traite d'abord par un agent de débromhydratation puis par un agent permettant d'éliminer le groupement protecteur du radical hydroxyle, pour obtenir le produit $I'_D$ recherché;

5) Pour préparer les produits de formule ($I'_E$)

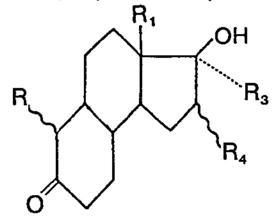

($I'_E$)

dans laquelle R, $R_1$, $R_3$ et $R_4$ ont la signification indiquée ci-dessus, étant entendu que lorsque $R_4$ représente un atome d'hydrogène

a) lorsque $R_3$ représente un atome d'hydrogène, un radical méthyle, éthyle, propargyle ou isobuténylе, R et $R_1$ ne peuvent pas représenter chacun un radical méthyle;

b) lorsque $R_3$ représente un atome d'hydrogène, R ne peut pas représenter un radical méthyle lorsque $R_1$ représente un radical éthyle et inversement, R ne peut pas représenter un radical éthyle lorsque $R_1$ représente un radical méthyle, on fait agir un réactif d'hydrogénation sur un produit de formule ($II'_E$):

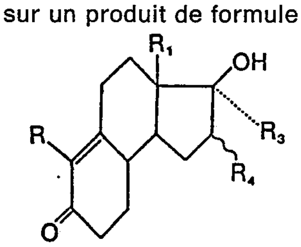

($II'_E$)

dans laquelle R, $R_1$, $R_3$ et $R_4$ ont la signification précédente,

6) Pour préparer les produits de formule ($I'_F$)

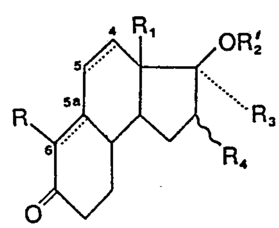

($I'_F$)

dans laquelle R, $R_1$, $R_3$, $R_4$ et les traits pointillés ont les significations indiquées ci-dessus et $R'_2$ a les significations de $R_2$ à l'exclusion de la valeur hydrogène, étant entendu que sont exclus les produits dans lesquels $R_4$ représente un atome d'hydrogène, le trait pointillé en position 4(5) n'indique pas la présence d'une seconde liaison et le trait pointillé en position 5a(6) indique la présence d'une seconde liaison et

a) $R_1$ représente un radical méthyle et $R_3$ représente un atome d'hydrogène et

α) $R_1$ représente un radical méthyle et $R'_2$ re-

présente un radical acétyle, benzoyle ou tert-butyle,

β) $R_1$ représente un radical éthyle et $R'_2$ représente un radical tert-butyle,

b) R représente un radical éthyle ou propyle, $R_1$ représente un radical méthyle, $R'_2$ représente un radical benzoyle et $R_3$ représente un atome d'hydrogène,

que sont exclus également les produits dans lesquels $R_4$ représente un atome d'hydrogène, les traits pointillés n'indiquent pas la présence de secondes liaisons et

a) R et $R_1$ représentent chacun un radical méthyle, $R_3$ représente un atome d'hydrogène et $R'_2$ représente un radical acétyle ou benzoyle,

b) $R_3$ représente un atome d'hydrogène, R et $R_1$ représentent chacun un radical méthyle et $R'_2$ représente un radical méthoxyméthyle,

c) R, $R_1$ et $R_3$ représente chacun un radical méthyle et $R'_2$ représente un radical acétyle,

soit l'on fait agir un dérivé fonctionnel de formule $R'_2$–E dans laquelle E représente le reste d'un groupement fonctionnel choisi parmi les halogénures d'alkyle, alkényle ou alkynyle ou les halogénures ou anhydrides mixtes ou symétriques d'acyles et $R'_2$ a la signification précédente, sur un produit de formule ($II_F$):

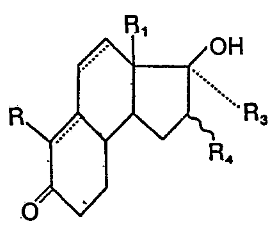

($II_F$)

dans laquelle R, $R_1$, $R_3$ et $R_4$ ont la signification précédente, étant entendu que les valeurs des substituants R, $R_1$, $R'_2$, $R_3$, $R_4$ ainsi que les traits pointillés sont soumis aux restrictions précédentes, pour obtenir un produit de formule (I'F) recherché,

soit, pour préparer les produits de formule ($I'_F$) dans laquelle le trait pointillé en position 4(5) ne représente pas une seconde liaison, on fait agir un produit de formule ($II'_F$):

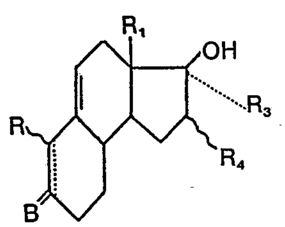

($II'_F$)

dans laquelle B a la signification précédente et R, $R_1$, $R_3$ et $R_4$ sont soumis aux restrictions précédentes, avec un dérivé fonctionnel de formule:

$R'_2$–E

dans laquelle $R'_2$ et E ont la signification précédente, pour obtenir un produit de formule ($III'_F$):

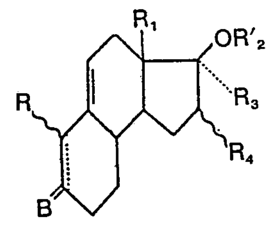

($III'_F$)

que l'on traite de manière à éliminer le radical protecteur de la fonction cétonique pour obtenir le produit de formule ($I'_F$) dans laquelle le trait pointillé en position 4(5) ne représente pas une seconde liaison.

La condensation du magnésien de formule ($III_A$) R $CH_2$ Mg X, qui est de préférence un bromure d'éthylmagnésium, sur le produit de formule ($II_A$) peut être effectuée dans les conditions habituelles de réaction du type organo magnésien. On opère de préférence dans un solvant anhydre tel que le tétrahydrofuranne ou l'éther éthylique; on opère de préférence à basse température et plus préférentiellement à une température de l'ordre de −50 °C.

La saponification des produits obtenus est effectuée par traitement par une base telle que la soude, la potasse ou la baryte. On isole le produit attendu par traitement final par un acide tel que l'acide chlorhydrique, acétique ou sulfurique.

Parmi les produits de formule ($II_A$) on préfère les produits dans lesquels A représente un radical acétyle.

Parmis les groupements protecteurs du groupement cétonique en position 7 du produit de formule ($III_B$) on préfère les groupements éthylène cétal, l'ène-amine pyrrolidinique, ou l'éther énolique, méthoxy ou éthoxy.

Les réactifs de protection du radical cétonique que l'on fait agir sur le produit de formule ($II_B$) sont alors respectivement l'éthylène glycol, la pyrrolidine, le méthanol ou l'éthanol. Lorsque l'on utilise l'éthylène glycol ou un mono alcool, on opère de préférence en présence d'un acide tel que l'acide paratoluène sulfonique.

L'agent d'oxydation avec lequel on traite le produit de formule ($III_B$) pour obtenir le produit de formule ($IV_B$) est de préférence le dichromate de pyridinium. On peut cependant utiliser d'autres réactifs tels que la solution sulfochromique, le chlorochromate de pyridinium, le trioxyde de chrome dans le pyridine ou un réactif d'oxydation d'Oppenauer tel que l'isopropoxyde ou le tert-butoxyde d'aluminium.

La base forte en présence de laquelle on fait agir l'iodure de formule $R'_4$ I sur le produit de formule ($IV_B$) est de préférence un amidure de lithium, en particulier le diisopropylamidure de lithium formé in situ à partir de diisopropylamine et de butyllithium. On peut cependant utiliser d'autres bases fortes telles que les amidures en général ou un hydrure tel que l'hydrure de sodium. La réduction du produit de formule ($V_B$) en produit de formule ($V'_B$) est effectuée de préférence à l'aide d'hydrure de lithium aluminium. On peut cependant utiliser d'autres hydrures tels que les borohydrures de sodium, de potassium ou de lithium.

La déprotection du produit de formule ($V'_B$) est effectuée selon les méthodes usuelles. On utilise de préférence l'hydrolyse acide. On peut, par exemple, traiter le produit par l'acide paratoluène sulfonique. On peut également utiliser les acides chlorhydrique ou acétique.

Les produits de formule ($V_B$) et ($V'_B$) peuvent se présenter sous forme d'un mélange d'isomères 2α et 2β ou sous forme d'un seul de ces produits. On peut séparer les mélanges selon les méthodes usuelles, par exemple, la chromatographie.

Lorsqu'un seul des isomères est obtenu de façon exclusive ou prépondérante, l'autre isomère peut généralement être obtenu après isomérisation de l'isomère unique isolé. On peut effectuer une telle isomérisation par chauffage en milieu basique, par exemple, dans une solution de potasse méthanolique. On sépare ensuite le mélange par les méthodes usuelles, de préférence la chromatographie. La séparation ou l'isomérisation est effectuée de préférence sur les produits de formule ($V_B$).

L'addition du magnésien $R_3$ Mg Hal, de préférence un bromure d'alkyl magnésium, sur le produit de formule ($II_C$), est effectué selon les méthodes usuelles, par exemple, dans un solvant tel que le tétrahydrofuranne ou l'éther éthylique.

La déprotection du radical cétonique est effectuée dans les conditions usuelles décrites ci-dessus.

La protection du radical hydroxyle du produit de formule ($II_D$) est effectuée de manière usuelle. On utilise de préférence de l'estérification et plus préférentiellement l'acétylation à l'aide, par exemple, de l'anhydride acétique, on peut également utiliser la protection par le tétrahydropyrannyle.

La formation de la double liaison en position 4,5 est effectuée de préférence par traitement à l'aide de la N-bromosuccinimide suivi de traitement par un agent de débromhydratation tel que la pyridine, la collidine ou le mélange bromure de lithium-carbonate de lithium.

L'élimination du groupement protecteur du radical hydroxyle est effectué selon les méthodes usuelles. Lorsque le radical hydroxyle est protégé par estérification à l'aide d'un radical acyle, par exemple acetyle, le groupement protecteur est éliminé par saponification dans les conditions décrites ci-dessus. Lorsque le radical hydroxyle est protégé par un tétrahydropyrannyle, l'élimination est effectuée par hydrolyse acide dans les conditions usuelles.

L'hydrogénation des produits de formule ($II'_E$) en produits de formule ($I'_E$) est effectuée par exemple par action de l'hydrogène en présence d'un catalyseur tel que le platine ou l'oxyde de platine.

Le réactif de formule $R'_2$–E que l'on fait agir soit sur le produit de formule ($II_F$) soit sur le produit de formule ($II'_F$), peut être un halogénure d'alkyle, d'alkényle ou d'alkynyle, ces radicaux pouvant être éventuellement interrompus par un hétéroatome. On préfère alors le chlorure ou le bromure et on opère de préférence en présence d'une base organique ou minérale ou d'un sel basique, tels que la triéthylamine ou le carbonate acide de sodium, potassium ou lithium.

On peut également faire agir un anhydride mixte ou symétrique formé à partir du radical acyle avec lequel on veut estérifier le radical hydroxyle. On peut citer par exemple les anhydrides acétique ou butyrique. On peut également faire agir sur les produits de formules ($II_F$) ou ($II'_F$) une halogénure d'acyle tel que le chlorure d'acétyle.

La déprotection des produits de formule ($III'_F$) est effectuée dans les conditions décrites précédemment.

La préparation des produits racémiques et de la série antipodale est effectuée de façon identique en partant des produits de départ correspondants.

La produits utilisés au départ du procédé ci-dessus peuvent être préparés comme suit:

Les produits de formule ($II_A$) peuvent être préparés selon les procédés décrits dans les brevets français 1 364 556 et 1 476 509.

Les produits de formule ($II_B$) comprennent d'une part des produits de formule ($I'_A$) tels que préparés par le procédé décrit dans la présente demande et d'autre part des produits décrits dans la référence suivante:

J. Chem. Soc. 1962 p 1312–13; BF 1 359 675;
Helv. Chim. Acta 1971, 54(7) p 2121–32;
J. Org. Chem. 1969 34(1) p 197–12.

Les produits de formule ($II_C$) comprennent d'une part les produits de formule ($V_B$) dont la préparation est décrite dans la présente demande et d'autre part des produits dans lesquels $R_4$ = H qui sont décrits dans le brevet français 1 553 958.

Les produits de formule ($II_D$) comprennent d'une part les produits de formule ($I'_C$) dont la préparation est donnée ci-dessus dans la présente demande et d'autre part un produit connu décrit dans le brevet belge BB 663 197.

Les produits de formule ($II'_E$) sont compris dans les produits de formule ($II_D$).

Les produits de formule ($II_F$) sont décrits précédemment.

Les produits de formule ($II'_F$) sont préparés de manière analogue aux produits de formule ($III_B$) décrits précédemment. On met en œuvre le même procédé au départ de produits de formule ($II_F$) dans laquelle le trait pointillé en position 4(5) ne représente pas une seconde liaison.

Les produits de formule (I) qui ne sont pas compris dans la formule (I'), pour laquelle un procédé de préparation est décrit dans la présente demande, sont connus. Ils peuvent être préparés selon les procédés décrits dans les références suivantes:

a) Produits dans lesquels existe une double liaison en position 5a(6) et dans lesquels $R_3$ représente un atome d'hydrogène et
   α) R représente un radical méthyle:
   - brevet français BF 1 359 675
   - J. Org. Chem, 1969, 34(5) p 1457–8
   - Ann. 669, p 153–9 (1963)
   - J. Org. Chem, 1969, 34 (1) p 107–12
   - Demande allemande OLS 1 811 693,

β) R représente un alkyle différent et méthyle:
- Brevet français BF 1 359 675
- Helv. Chim. Acta, 1971, 54(7) p 2121–32,

b) Produit dans lequel existe une double liaison en position 5a(6) et dans lequel $R_3$ est différent d'hydrogène:
- Brevet belge BB 663 197,

c) Produits saturés dans lesquels $R_3$ représente un atome d'hydrogène et $R_1$ représente un radical méthyle:
- Brevet belge BB 663 197
- J. Chem. Soc. 1949 p 1855–65
- J. Chem. Soc. 1962 p 1312–13
- Brevet américain USP 3 956 316
- Brevet américain USP 3 644 429,

d) Produit saturé dans lequel $R_3$ représente un atome d'hydrogène et $R_1$ représente éthyle:
- Brevet belge 767 347

e) Produits saturés dans lesquels $R_3$ est différent d'hydrogène et $R_1$ représente un radical méthyle:
- Brevet belge BB 663 197
- Brevet américain USP 3 496 199

Dans les exemples suivants et dans tout le brevet, la nomenclature adoptée est dérivée du 7-H benz (E) indène de formule:

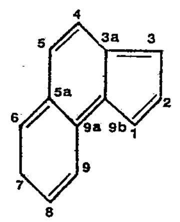

(forme tautomérique de la formule)

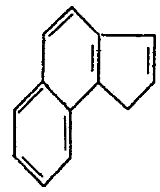

Les exemples suivants illustrent l'invention sans toutefois la limiter.

Exemple 1:

2α, 3aβ,6-triméthyl, 3β-hydroxy 1,2,3,3a,4,5,8,9,9a, 9b-décahydro 7-H-benz(e) inden-7-one.

Stade A:

3a, 6-diméthyl, 3β-hydroxy 7,7-éthylène dioxy 1,2,3,3a,4,6,8,9,9a,9b-décahydro 7-H benz (e) inden-7-one.

On chauffe pendant 3 heures à reflux sous azote un mélange de 5,5 g 3a, 6-diméthyl 3β-hydroxy 1,2,3,3a,4,5,8,9,9a,9b-décahydro 7-H-benz (e) inden-7-one, 5,5 $cm^3$ d'éthylène glycol, 9 $cm^3$ d'orthoformiate d'éthyle et 200 mg d'acide paratoluène sulfonique dans 200 $cm^3$ de benzène. On ajoute du carbonate de sodium anhydre, filtre, lave à la soude et évapore à sec. On chromatographie sur silice en éluant avec un mélange acétone-hexane (1–9). On obtient 3,22 g de produit attendu.

Stade B:

3aβ, 6-diméthyl 7,7-éthylène dioxy, 1,2,3,3a,4,6,8, 9,9a,9b-décahydro 7H-benz (e) inden 3,7-dione.

1,9 g du produit obtenu au stade précédent est oxydé à l'aide de 6,37 g de dichromate de pyridinium dans 19,5 $cm^3$ d'un mélange chlorure de méthylène-diméthylformamide à température ambiante pendant 2 heures. On reprend à l'eau, extrait à l'éther et recristallise dans l'hexane. On obtient 1,235 g de mélange d'épimères F = 95 °C RMN 0,85 ppm (méthyle en 3a).

Stade C:

2α, 3aβ, 6-triméthyl 7,7-éthylène dioxy 1,2,3,3a,4, 6,8,9,9a,9b décahydro 7 H-benz (e) inden-3,7-dione.

On prépare à 0 °C et sous azote une solution de diisopropylamidure de lithium à partir de 0,17 $cm^3$ de diisopropylamine et de 0,78 $cm^3$ d'une solution hexanique 1,6M de butyl-lithium dans 2 $cm^3$ de tétrahydrofuranne en présence d'un cristal de bipyrydil comme indicateur coloré. Après 10 minutes on ajoute à cette solution rouge à 0 °C, 276 mg de produit obtenu au stade B dans 1 $cm^3$ de tétrahydrofuranne et laisse revenir à température ambiante. Après 20 minutes, on ajoute 0,4 $cm^3$ d'iodure de méthyle, la couleur devient jaune. Un précipité apparait rapidement. On verse dans l'eau, extrait à l'éther et obtient 294 mg de produit attendu F = 110 °C. Après recristallisation dans un mélange hexane-éther, on obtient 225 mg de produit attendu F = 136 °C.

RMN: 0,87 ppm (méthyle en 3a)

Stade D:

2α , 3a β 6-triméthyl 3β-hydroxy 7,7-éthylène dioxy 1,2,3,3a,4,6,8,9,9a,9b-décahydro 7-H benz(e) inden-7-one.

On fait réagir 130 mg de produit obtenu au stade précédent avec 32 mg d'hydrure de lithium aluminium dans l'éther à température ambiante. Après 10 minutes, on hydrolyse par l'eau et la soude à 15%, filtre, évapore le filtrat et obtient le produit attendu sous forme d'huile.

RMN: 0,72 ppm (méthyle en 3a)

Stade E:

2α, 3aβ,6-triméthyl, 3β-hydroxy 1,2,3,3a,4,5,8,9,9a, 9b-décahydro 7-H-benz (e) inden-7-one.

Le produit obtenu au stade précédent est placé au reflux dans l'acétone aqueuse en présence de quelques milligrammes d'acide paratoluène sulfonique. On refroidit, extrait à l'éther, et chromatographie sur silice en éluant avec un mélange acétone-hexane (2–8). Aprés recristallisation dans le méthanol aqueux, on obtient 60 mg de produit attendu F = 139 °C.

Pouvoir rotatoire $[\alpha]^{25}_D = -51°$ (c = 1% dans le chloroforme)

RMN: 0,90 ppm (méthyle en 3a)

Analyse: $C_{16}$ $H_{24}$ $O_2$

Calculé: C% 77,37 H% 9,74

Trouvé: C% 77,1 H% 9,6

Exemple 2:
2β, 3aβ,6-triméthyl 3β-hydroxy 1,2,3,3a,4,5,8,9,9a, 9b-décahydro 7H-benz (e) inden-7-one.
Stade A:
2β, 3aβ,6-triméthyl 7,7-éthylène dioxy 1,2,3,3a,4,6, 8,9,9a,9b-décahydro 7H-benz (e) inden-3,7-dione.

On chauffe à reflux pendant 90 minutes 240 mg de produit obtenu au stade C de l'exemple 1 dans 2,6 $cm_3$ de potasse méthanolique 2N. On évapore à sec, reprend par l'eau et extrait à l'éther. On obtient un mélange (1–1) de produits 2α et 2β que l'on chromatographie sur colonne de silice en éluant avec un mélange acétate d'éthyle-hexane (1–9). On obtient 110 mg de produit attendu (huile). RMN: 0,8 ppm (méthyle en 3a).

Stade B:
2β, 3aβ,6-triméthyl 3β-hydroxy 7,7-éthylène dioxy 1,2,3,3a,4,6,8,9,9a,9b-décahydro 7-H-benz (e) inden-7-one.
On opère comme indiqué au stade D de l'exemple 1 au départ de 110 mg de produit obtenu au stade précédent.

Stade C:
2β, 3aβ,6-triméthyl 3β-hydroxy 1,2,3,3a,4,5,8,9,9a, 9b-décahydro 7-H-benz (e) inden-7-one.
On opère comme indiqué au stade E de l'exemple 1 au départ du produit obtenu au stade précédent. On obtient 70 mg de produit attendu qui après recristallisation dans un mélange éther-hexane donne un produit pur F = 81 °C.
Pouvoir rotatoire $[\alpha]^{25}_D = -32°$ (c = 1% dans le chloroforme)
Analyse: $C_{16} H_{24} O_2$
Calculé: C% 77,37 H% 9,74
Trouvé: C% 77,2 H% 9,8

Exemple 3:
3aβ,6-diéthyl 3β-hydroxy 1,2,3,3a,4,5,8,9,9a,9b-décahydro 7-H-benz (e) inden-7-one.
On dissout 500 mg de δ lactone de 1 β-acétoxy 4-(2′-carboxyéthyl) 5-hydroxy 7a-éthyl 3a, 4β, 7,7a-tétrahydroindane dans 3,8 $cm^3$ de tétrahydrofuranne anhydre et ajoute lentement et à −60 °C, 3,36 $cm^3$ d'une solution éthérée (0,8M) de bromure de n-propyl magnésium fraîchement préparée.

Aprés une heure à −60 °C, on détruit l'excès de magnésium à l'eau. On évapore le tétrahydrofuranne, ajoute une solution saturée de chlorure d'ammonium, extrait à l'éther et sèche. Après évaporation de l'éther, on chauffe de résidu obtenu à reflux pendant une heure dans 3,7 $cm^3$ de potasse méthanolique 2N. On neutralise à l'acide acétique, évapore à sec, reprend à l'eau et extrait au chlorure de méthylène. On obtient 470 mg de produit brut que l'on recristallise dans le mélange acétone-hexane. On obtient 100 mg de produit pur F = 131,5 °C.
Pouvoir rotatoire $[\alpha]^{25}_D = -58°$ (c = 1% dans le méthanol)
Analyse: $C_{17} H_{26} O_2$
Calculé: C% 77,82 H% 9,99
Trouvé: C% 78,0 H% 10,0

Exemple 4:
3a β-éthyl 6-propyl 3β-hydroxy 1,2,3,3a,4,5,8,9,9a, 9b-décahydro 7-H-benz (e) inden-7-one.
On opère comme indiqué à l'exemple 3 au départ de 500 mg de δ-lactone de 1β-acétoxy 4-(2′-carboxyéthyl) 5-hydroxy 7a-éthyl 3a, 4β, 7, 7a-tétrahydroindane et en utilisant du bromure de n-butylmagnésium. On obtient 220 mg de produit brut attendu que l'on recristallise dans l'acétone. On obtient 105 mg de produit pur F = 127 °C.
Pouvoir rotatoire $[\alpha]^{25}_D = -55°$ (c = 1% dans le méthanol)
Analyse: $C_{18} H_{28} O_2$
Calculé: C% 78,21 H% 10,21
Trouvé: C% 78,4 H% 10,1

Exemple 5:
3a β-éthyl 6-méthyl 3β-acétoxy 1,2,3,3a,4,5,8,9,9a, 9b-décahydro 7-H-benz (e) inden-7-one.
On fait agir de l'anhydride acétique sur la 3a β-éthyl 6-méthyl 3β-hydroxy 1,2,3,3a,4,5,8,9,9a,9b-décahydro 7-H benz (e) inden-7-one dans la pyridine. On recristallise dans le méthanol aqueux et obtient le produit attendu F = 99 °C.

Exemple 6:
3a β-éthyl, 6-méthyl, 3β-hydroxy 1,2,3,3a,8,9,9a, 9b-octahydro 7-H-benz (e) inden-7-one.
Stade A:
3a β-éthyl 5-bromo 6-méthyl 3β-acétoxy 1,2,3,3a,4,5,8,9,9a,9b-décahydro 7-H-benz (e) inden-7-one.
On laisse au contact pendant 16 heures un mélange de 210 mg de produit préparé à l'exemple 5, 0,72 $cm^3$ d'anhydride acétique et 10 mg d'acide paratoluène sulfonique. On ajoute 0,3 $cm^3$ d'acide acétique et 0,43 $cm^3$ d'eau. Après deux heures on refroidit à +5 °C, ajoute 130 mg de N-bromosuccinimide et agite deux heures. On ajoute 0,72 $cm^3$ d'eau et filtre les cristaux. On obtient 190 mg de produit attendu. F ≃ 120 °C.

Stade B:
3aβ-éthyl 6-méthyl 3β-hydroxy 1,2,3,3a,8,9,9a,9b-octahydro 7-H-benz (e) inden-7-one.
Sous courant d'azote on distille pendant 30 minutes presque à sec 4,5 $cm^3$ de diméthylformamide qui contiennent un mélange de 190 mg de produit obtenu au stade précédent, 95 mg de bromure de lithium et 47 mg de carbonate de lithium. On refroidit, ajoute 5 $cm^3$ d'eau, 0,1 $cm^3$ d'acide acétique, verse dans l'eau et extrait au chlorure de méthylène. Après saponification on obtient 128 mg de produit attendu que l'on chromatographie sur silice (éluant acétone-hexane 15–85) et recristallise dans un mélange acétone-hexane F = 155 °C.
Pouvoir rotatoir $[\alpha]^{25}_D = -207°$ (1% dans le chloroforme)
Analyse: $C_{16} H_{22} O_2$, $0,5C_3 H_6O$ (acétone)
Calculé: C% 76,32 H% 9,15
Trouvé: C% 76,65 H% 9,3

Exemple 7:
Butanoate de 6-éthyl 3aβ-méthyl 7-oxo 1,2,3,3a,4,5,8,9,9a,9b-décahydro 1H-benz (e) inden-3-yle.

On dissout 0,75 g de 3a β-méthyl 6-méthyl 3β-hydroxy 1,2,3,3a,4,5,8,9,9a,9b-décahydro 7-H-benz (e) inden-7-one dans 1,5 $cm^3$ d'anhydride butyrique et 1,05 $cm^3$ de triéthylamine sèche. On ajoute 30 mg de 4-diméthylamino pyridine. On agite deux heures à température ambiante. On verse dans une solution saturée de carbonate acide de sodium et agite deux heures. Le produit est extrait par le chlorure de méthylène. On lave à l'eau, sèche et évapore le solvant. On recueille 0,96 g d'une huile que l'on chromatographie sur silice (éluant benzène – acétate d'éthyle 9–1). On obtient le produit attendu.

Analyse: $C_{20} H_{30} O_3$
Calculé: C% 75,43 H% 9,49
Trouvé: C% 75,6 H% 9,50
Spectre RMN: $CDCl_3$ ppm

| | |
|---|---|
| 0,9 ppm (t) | H du méthyle d'un groupement $\underline{CH_3}$–$CH_2$ |
| 0,97 ppm | H du méthyle en 3a |
| 0,97 ppm (t) | H du méthyle d'un groupement $\underline{CH_3}$–$CH_2$ |
| 4,68 ppm (t) | proton en 3α (J = 8 Hz) |

Exemple 8:
6-éthyl 3-(méthoxyméthoxy)3aβ-méthyl 1,2,3,3a,4,5,8,9,9a,9b-décahydro 7-H-benz (e) inden-7-one.

2 g de 3a-méthyl, 6-éthyl 3β-hydroxy 1,2,3,3a,4,5,8,9,9a,9b décahydro 7-H benz (e) inden-7-one sont mis en solution dans 20 $cm^3$ de diméthylformamide sec puis on ajoute à température ambiante 1 g de carbonate de lithium et 1 $cm^3$ de chlorure de méthoxyméthyle. On ajoute successivement 1 g de carbonate de lithium et 1 $cm^3$ de chlorure de méthoxyméthyle après deux heures, quatre heures de cinq heures de réaction, on rajoute enfin 1 $cm^3$ de chlorure de méthoxyméthyle après six heures. Après sept heures, on verse le mélange dans 40 $cm^3$ d'une solution saturée de carbonate acide de sodium et 80 $cm^3$ d'eau. On extrait à l'acétate d'éthyle, lave à l'eau, évapore le solvent. On obtient 2,4 g d'une huile que l'on purifie sur silice (éluant benzène–acétate d'éthyle 7–3). On obtient 1,6 g de produit attendu Rf = 0,48.

Analyse: $C_{18} H_{28} O_3$
Calculé: C% 73,93 H% 9,65
Trouvé: C% 73,9 H% 9,7
Spectre RMN: $CDCl_3$

| | |
|---|---|
| 0,9 ppm (t) | H du $\underline{CH_3}$–$CH_2$ en 6 |
| 0,06 ppm | H du méthyle en 3a |
| 3,58 ppm (t) | proton en 3 |
| 4,06 ppm | $\underline{CH_3}$–O–$CH_2$ |
| 4,68 ppm | $CH_3$–O–$\underline{CH_2}$ |

Exemple 9:
6-éthyl 3a β-méthyl 3-(2-propenyloxy) 1,2,3,3a,4,5,8,9,9a,9b décahydro 7-H-benz (e) inden-7-one.

Stade A:
1,2,3,3a,4,6,8,9,9a,9b décahydro 6β-éthyl 3a β-méthyl spiro /7H-benz-(e) indène 7,2′-/1,3/ dioxolanne/ 3β-ol.

On met en suspension 4 g de 6β-éthyl 3a-méthyl 3β-hydroxy 1,2,3,3a,4,5,8,9,9a,9b décahydro 7-H-benz (e) inden-7-one dans 40 $cm^3$ d'éthylène glycol et 4 $cm^3$ d'orthoformiate d'éthyle. On chauffe à 75° et ajoute en une fois 20 mg d'acide paratoluène sulfonique. Après 3 heures en rajoute 20 mg d'acide paratoluène sulfonique et l'on poursuit le chauffage une heure. On refroidit et après avoir ajouté 1 $cm^3$ de triéthylamine on verse sur 80 $cm^3$ d'eau.

On extrait à l'acétate d'éthyle puis lave la phase organique à l'eau. On sèche, concentre à sec et obtient 4,6 g d'une résine. On chromatographie sur silice en éluant par un mélange dyclohexane–acétate d'éthyle (5–5). On obtient 3,4 g de produit cherché F = 148 °C.

Stade B:
6β-éthyl 3a β-méthyl 3-(2-propényloxy) 1,2,3,3a,4,6,8,9,9a,9b-décahydro 7,7-éthylène dioxy /7H-benz (e) indène-7-one.

On dissout 3 g de produit obtenu au stade A dans 30 $cm^3$ de tétrahydrofuranne. On ajoute 0,489 g d'hydrure de sodium à 50% dans l'huile.

Le mélange est plongé trente minutes dans un bain d'huile à 45 °C puis l'on ajoute 3 $cm^3$ de bromure d'allyle. On maintient le chauffage 3 heures à 45 °C. On rajoute alors 3 $cm^3$ de bromure d'allyle et l'on chauffe à nouveau 3 heures à 45° puis une heure au reflux. On refroidit, verse sur 300 $cm^3$ d'une solution saturée de carbonate acide de sodium. Après extraction à l'acétate d'éthyle, on lave à l'eau, sèche et amène à sec. On recueille 3,5 g d'une huile dont on chromatographie 250 mg sur silice en éluant avec un mélange benzène–acétate d'éthyle (7–3). On recueille 0,16 g de produit attendu.

Spectre RMN: $CDCl_3$

| | |
|---|---|
| 0,76 ppm | H du méthyle en 3a |
| 0,81 ppm (t) | H du méthyle terminal en 6 (J = 7 Hz) |
| 3,5 ppm | proton en 3α |
| 4 ppm (m) | O–$\underline{CH_2}$–CH = $CH_2$ |
| 5,66 ppm (m) | –O $CH_2$ $\underline{CH}$ = $CH_2$ |

Stade C:
6-éthyl 3aβ méthyl 3-(2-propényloxy) 1,2,3,3a,4,5,8,9,9a,9b-décahydro 7-H-benz (e) inden-7-one.

3 g de produit obtenu au stade B sont mis en solution dans 18 $cm^3$ d'acide chlorhydrique aqueux 2N et 450 $cm^3$ de méthanol. On agite 4 heures à température ambiante puis évapore le méthanol et le résidu est repris par 45 $cm^3$ d'eau. Après extraction à l'acétate d'éthyle, on lave avec une solution aqueuse de carbonate acide de sodium puis à l'eau. On sèche et évapore les solvants. On obtient 2,5 g d'une huile que l'on chromatographie sur silice avec un éluant constitué de benzène–acétate d'éthyle (7–3). On obtient 1,64 g de produit cherché.

Analyse: $C_{19} H_{28} O_2$
Calculé: C% 79,12 H% 9,78
Trouvé: C% 79,3 H% 9,9
Spectre RMN $CDCl_3$

| | |
|---|---|
| 0,91 ppm (t) | H du méthyle terminal en 6 (J = 7 Hz) |
| 0,96 ppm | H du méthyle en 3a |
| 3,43 ppm (t) | proton en 3α |
| 5,26 ppm (m) | –CH = $\underline{CH_2}$ |
| 5,98 ppm (m) | –$\underline{CH}$ = $CH_2$ |

Exemple 10:

3a β méthyl 6-propyl 3β-acétoxy 1,2,3,3a,4,5,8,9,9a, 9b-décahydro 7-H-benz (e) inden-7-one.

On dissout 1,35 g de γ-lactone de 1β-acétoxy 4-(2'-carboxyéthyl)-5-hydroxy 7a-β-méthyl 3aα, 4β,7,7a-tétrahydroindane dans 10 $cm^3$ de tétrahydrofuranne anhydre. On ajoute en 30 minutes et à −60 °C, 9,6 $cm^3$ d'une solution éthérée (0,8M) de bromure de n-butyl magnésium fraîchement préparé. Après une heure à −60 °C, on détruit l'excès de magnésien par 15 $cm^3$ d'eau.

On évapore le solvant, ajoute une solution saturée de chlorure d'ammonium, extrait à l'éther et sèche. Après évaporation de l'éther on obtient 1,43 g de produit attendu.

Exemple 11:

3aβ6-méthyl 3β-acétoxy 1,2,3,3a,8,9,9a,9b-octahydro 7-H-benz (e) inden-7-one.

Stade A:

3aβ 6-diméthyl 5-bromo 3β-acétoxy 1,2,3,3a,4,5,8, 9,9a,9b-décahydro 7-H benz (e) inden-7-one.

On agite 23,4 g de 3a β 6-diméthyl 3β-hydroxy 1,2,3,3a,4,5,8,9,9a,9b-décahydro 7-H benz (e) inden-7-one dans 100 $cm^3$ d'anhydride acétique avec 1 g d'acide paratoluène sulfonique. On abandonne ensuite 16 heures à 20 °C environ. On ajoute 40 $cm^3$ d'acide acétique et 60 $cm^3$ d'eau. On refroidit pour ne pas dépasser 30 °C. Après deux heures on refroidit à +5 °C et introduit en 10 minutes sans dépasser 15 °C, 17,8 g de N-bromo succinimide. On agite une demi-heure, puis ajoute 100 $cm^3$ d'eau. On essore, lave à l'éthanol à 40% d'eau, puis avec de l'éthanol. On sèche à 40 °C et obtient 26,3 g de produit attendu.

On recristallise dans un mélange chlorure de méthylène–éther isopropylique F = 140 °C.

$[\alpha]^{25}_D = +226° \pm 3°$ (c = 1% dans le chloroforme).

Analyse: $C_{17}H_{23}O_3Br$

Calculé: C% 57,47 H% 6,52

Trouvé: C% 57,8 H% 6,7

Stade B:

3aβ6-diméthyl, 3β-acétoxy 1,2,3,3a,8,9,9a,9b-octahydro 7-H-benz (e) inden-7-one.

On mélange 2 g de bromure de lithium, 1 g de carbonate de lithium et 90 $cm^3$ de diméthylformamide. On distille 10 $cm^3$ pour sécher et ajoute 4 g de produit obtenu au stade A. On distille environ 50 $cm^3$ en une demi-heure et ajoute après refroidissement 100 $cm^3$ d'eau puis 2 $cm^3$ d'acide acétique. On verse dans l'eau, extrait trois fois au chlorure de méthylène, lave à l'eau, sèche, distille à sec. On chromatographie sur silice (solvant chlorure de méthylène à 5% d'éther isopropylique). On recristallise le produit dans l'éther de pétrole et obtient 2,32 g de produit attendu F = 75 °C.

$[\alpha]^{25}_D = -143° \pm 1,5°$ (c = 1% dans le chloroforme).

Analyse: $C_{17}H_{22}O_3$

Calculé: C% 74,42 H% 8,08

Trouvé: C% 74,5 H% 8,2

Exemple 12:

3aβ6-diméthyl 3β-hydroxy 1,2,3,3a,8,9,9a,9b-octahydro 7-H-benz (e) inden-7-one.

A 15,2 g de produit préparé à l'exemple 11, on ajoute 152 $cm^3$ d'éthanol et 55 $cm^3$ de soude aqueuse 2N. On laisse deux heures à 20 °C, ajoute lentement 600 $cm^3$ d'eau, essore le produit cristallisé, lave à l'eau, sèche et obtient 11,6 g de produit. On dissout dans le chlorure de méthylène, traite au charbon actif, ajoute de l'éther isopropylique, concentre sous pression réduite à 30 °C environ, essore, lave à l'éther isopropylique, sèche et obtient 11,3 g de produit attendu F = 156 °C.

$[\alpha]^{25}_D = -188° \pm 2°$ (c = 1% dans le chloroforme)

Analyse: $C_{15}H_{20}O_2$

Calculé: C% 77,55 H% 8,68

Trouvé: C% 77,5 H% 8,7

Exemple 13:

3β-acétoxy 6-éthyl 3aβ-méthyl, 1,2,3,3a,4,5,8,9,9a, 9b-décahydro 7-H-benz (e) inden-7-one.

On dissout dans 24 $cm^3$ pyridine, 6 g de 3-hydroxy 6-éthyl 3a-méthyl 1,2,3,3a,4,5,8,9,9a,9b-décahydro 7-H-benz (e)-inden-7-one puis ajoute rapidement 12 $cm^3$ d'anhydride acétique pur. On maintient la solution en contact à température ambiante pendant 15 heures. La solution est versée dans 240 $cm^3$ d'eau et le précipité formé ets filtré, puis lavé à l'eau. Après séchage, on obtient 6,8 g de cristaux. Les cristaux sont recristallisés dans l'hexane. On obtient 6,5 g de produit pur F = 90 °C.

Analyse: $C_{18}H_{26}O_3$

Calculé: C% 74,44 H% 9,02

Trouvé: C% 74,4 H% 9,1

Spectre RMN ($CDCl_3$) ppm

| | |
|---|---|
| 0,91 (t) | H du méthyle terminal en position 6, J = 8 Hz |
| 0,97 | H du méthyle en 3a |
| 2,06 | $\underline{CH_3}CO$ |
| 4,65 (t) | proton en 3α J = 8 Hz. |

Exemple 14:

3β-propionyloxy 6-éthyl 3aβ-méthyl 1,2,3,3a,4,5,8, 9,9a,9b-décahydro 7H-benz (e) inden-7-one.

On opère comme indiqué à l'exemple 13, au départ de 1 g de produit 3-hydroxy et de 2 $cm^3$ d'anhydride propionique. Le produit est extrait à l'éther, lavé à l'aide d'une solution aqueuse saturée de bicarbonate de sodium et à l'eau puis séché et chromatographié sur silice en éluant au mélange benzène–acétate d'éthyle (9–1). On obtient 1,08 g de produit attendu. F = 50 °C.

$[\alpha]_D = -28° \pm 2°$ (c = 0,4% éthanol).

Analyse: $C_{19}H_{28}O_3$

Calculé: C% 74,96 H% 9,27

Trouvé: C% 75,0 H% 9,2

Exemple 15:

3β-valéryloxy 6-éthyl 3aβ-méthyl 1,2,3,3a,4,5,8,9, 9a,9b-décahydro 7H-benz (e) inden-7-one.

On opère comme indiqué à l'exemple 14, au départ de 0,5 g de produit 3-hydroxy et de 1 $cm^3$

d'anhydride valérique. On obtient 0,590 g de produit attendu.
$[\alpha]_D = -18° \pm 2°$ (c = 0,5% éthanol).
Analyse: $C_{21} H_{32} O_3$
Calculé: C% 75,86 H% 9,70
Trouvé: C% 76,0 H% 9,9

Exemple 16:
3β-hexanoyloxy 6-éthyl 3aβ-méthyl 1,2,3,3a,4,5,8,9,9a,9b-décahydro 7H-benz (e) inden-7-one.

On opère comme indiqué à l'exemple 14, au départ de 1 g de produit 3-hydroxy et de 2 $cm^3$ d'anhydride caproïque. On extrait le produit à l'acétate d'éthyle. On obtient 0,7 g de produit attendu.
$[\alpha]_D = -15,5°$ (c = 0,5% éthanol).
Analyse: $C_{22} H_{34} O_3$
Calculé: C% 76,25 H% 9,89
Trouvé: C% 76,3 H% 9,9

Exemple 17:
3β-(pyridin-3-yl) carbonyloxy 6-éthyl 3aβ-méthyl 1,2,3,3a,4,5,8,9,9a,9b-décahydro 7H-benz (e) inden-7-one.

Dans 15 $cm^3$ de pyridine anhydre, on ajoute sous agitation et sous gaz inerte, 1,95 g de chlorhydrate de chlorure de l'acide nicotinique. On ajoute ensuite 2,48 g de 3-hydroxy 6-éthyl 3aβ-méthyl 1,2,3,3a,4,5,8,9,9a,9b-décahydro 7H-benz (e) inden-7-one puis chauffe à 90–95 °C pendant 2 heures 30. On refroidit, verse dans un mélange eau-glace, filtre le précipité, le lave à l'eau et le sèche. Le filtrat est extrait à l'acétate d'éthyle. On lave la phase organique à l'eau, la sèche, évapore le solvant et joint le résidu au produit précédemment obtenu. On chromatographie sur silice en éluant au mélange chloroforme–méthanol (98–2) et obtient 2,75 g de produit attendu. F = 134 °C.
$[\alpha]_D = +46,5° \pm 2°$ (c = 0,75% éthanol)
Analyse: $C_{22} H_{27} NO_3$
Calculé: C% 74,75 H% 7,70 N% 3,96
Trouvé: C% 74,9 H% 7,8 N% 3,9

Exemple 18:

On a préparé une pommade pour application cutanée de composition:
– Produit de l'exemple 3 50 mg
– excipient q.s.p. 1 g
excipient: huile minérale, propylène, glycol, pétrolatum[(1)]
(1) Pétrolatum désigne un mélange d'hydrocarbures semisolides obtenus à partir du pétrole, voir par exemple USP XX;

Exemple 19:

On a préparé un gel de composition:
– Produit de l'exemple 3 50 mg
– excipient q.s.p. 1 g
excipient: alcool, carbopol, propylène, glycol, diisopropanolamine, eau.

Exemple 20:

On a préparé des gélules de composition:
– Produit de l'exemple 3 50 mg
– excipient q.s.p. 400 mg
excipient: stéarate de magnésium, lactose, amidon.

Etude pharmacologique des produits de l'invention.

Dans les tests suivants, le produit dénommé produit A est le 3aβ 6-diméthyl, 3β-hydroxy 1,2,3,3a,4,5,8,9,9a,9b-décahydro 7-H benz (e) inden-7-one décrite dans le brevet belge BB 663 197.

Le produit B est la 3aβ-méthyl 6-éthyl 3β-hydroxy 1,2,3,3a,4,5,8,9,9a,9b-décahydro 7-H benz (e) inden-7-one décrite dans le brevet français 1 359 675.

Le produit C est la 3aβ-éthyl 6-méthyl 3β-hydroxy 1,2,3,3a,4,5,8,9a,9b-décahydro 7-H-benz (e) inden-7-one décrite dans J. Org. chem. 1969, 34 (1) 107–12.

Les produits A, B et C sont des produits de formule (I).

1) Mesure de l'interaction avec le récepteur androgène.

Des prostates de rats adultes (200 g) castrés depuis 24 heures sont homogénéisées à 0–4 °C dans du Tampon T.S. (Tris 10 mM, saccharose 0,25 M, pH: 7,4) à raison de 1 g de prostate dans 5 ml de tampon. L'homogénat est centrifugé à 105 000 g × 60 min dans une ultracentrifugeuse. Le surnageant obtenu est appelé cytosol.

Des prélévements de 125 µl de cytosol sont incubés pendant 30 minutes ou vingt-quatre heures à 0 °C en présence de 5 nM de (3H) Testostérone en absence (Bo) ou en présence (B) de concentrations croissantes des produits radioinertes à tester. Après incubation, 100 µl de l'incubat sont agités avec 100 µl d'une suspension charbon-Dextran (charbon Norit A : 1,25% – Dextran T80 : 0,625%) pendant 10 minutes à 0–4 °C, dans une plaque microtitre, puis centrifugés à 800 g × 10 minutes à 0–4 °C.

La radiocactivité contenue dans les prélèvements de 100 µl de surnageant est alors mesurée apr scintillation liquide. La valeur du rapport B/Bo est représentée graphiquement en fonction de la concentration en produit radioinerte ajoutée. La concentration en produit à tester nécessaire pour inhiber de 50% la fixation de (3H) testostérone ($CI_{50}$) est déterminée graphiquement. Le rapport:

$$\frac{CI_{50} \text{ testostérone}}{CI_{50} \text{ produit}} \times 100$$

donne la valeur de l'affinité relative du produit à tester pour le récepteur androgène.

Des études antérieures ont permis de montrer qu'un produit dont l'affinité relative diminue lorsqu'on augmente le temps d'incubation est une anti-hormone potentielle. (Voir en particulier TIPS – Août 1980 p 324 et Advances in pharmacology and therapeutics vol 1 receptors p 259 1979).

Résultats:

| | Produit A | Produit B | Produit C | Produit de l'ex 1 | Produit de l'ex 2 | Produit de l'ex 3 | Produit de l'ex 4 | Produit de l'ex 12 |
|---|---|---|---|---|---|---|---|---|
| Affinité relative avec une incubation de 30 minutes en % | 46 | 7,2 | 11,4 | 7,2 | 17 | 9,7 | 2,3 | 73 |
| Affinité relative avec une incubation de 24 heures en % | 4 | 0,8 | 5 | 1,3 | 1,7 | 1,2 | 0,3 | 8,4 |

2) Etude in vivo sur l'organe costovertébral du hamster

a) Des hamsters dorés mâles, pesant 100–110 g et castrés depuis 7 jours, reçoivent en injonction sous cutanée, 125 µg par jour et par animal de propionate de testostérone dans 0,2 $cm^3$ d'une solution d'huile de sésame à 0,5% d'alcool benzylique. Une partie des animaux reçoit en plus un traitement par application topique, sur l'organe costovertébral droit, du produit à tester dissous dans l'éthanol. L'organe costovertébral gauche reçoit seulement de l'éthanol. Le traitement dure 8 jours. Vingt-quatre heures après ce dernier traitement les organes costovertébraux et la prostate sont prélévés et pesés.

Les animaux témoins ne recoivent que les solvants.

On obtient les résultats suivants exprimés en poids (mg).

| Traitements quotidiens | Glande témoins | Glande traitée (droite) | Prostate |
|---|---|---|---|
| Témoins | 6,3±0,7 | 6,4±0,5 | 17,8±2,7 |
| Propionate de testostérone 0,125 µg/J | 35,8±5,0 | 33,91±2,9 | 79,5±5,5 |
| Propionate de testostérone 0,125 µg/J + Produit B 1 mg/J | 41,8±5,2 | 17,1±2,2 | 80,1±9,3 |

Le produit B, en application locale sur l'organe costovertébral droit, entraîne un pourcentage d'inhibition de l'augmentation pondérale de cet organe de 61%.

Par contre, le produit B n'antagonise pas l'effet du propionate de testostérone sur la glande non traitée pas plus que sur la prostate, indiquant que ce produit n'a pas d'effet systémique.

b) On réalise la même étude avec des hamsters femelles, pour tester l'activité antiandrogène des produits des exemples 7 et 13 à la dose de 5 mg/jour.

On obtient les résultats suivants exprimés en poids (mg).

| Traitements quotidiens | Glande témoin | Glande traitée (droite) |
|---|---|---|
| Témoin | 3,0±0,4 | 3,4±0,4 |
| Propionate de testostérone 0,125 µg/J | 15,8±1,0 | 14,9±1,1 |
| Propionate de testostérone 0,125 µg/J +Produit de l'exemple 7 5 mg/J | 9,5±0,8 | 4,5±0,4 |
| Propionate de testostérone 0,125 µg/J +Produit de l'exemple 13 5 mg/J | 10,8±2,1 | 3,9±0,4 |

Le produit de l'exemple 7, en appllication locale sur l'organe costovertébral droit entraîne un pourcentage d'inhibition de l'augmentation pondérale de cet organe de 90%.

Le produit de l'exemple 13 entraîne une inhibition de 95,6%.

c) On réalise la même étude avec des hamsters femelles pour tester l'activité antiandrogène des produits des exemples 13 à 17 à la dose de 1 mg/jour.

On obtient les résultats suivants exprimés en poids (mg).

| Traitements quotidiens | Glande témoin | Glande traitée (droite) | Pourcentage d'inhibition de l'augmentation pondérale |
|---|---|---|---|
| Témoin | 3,3±0,6 | 3,3±0,6 | |
| Propionate de testostérone 0,125 µg/j | 11,0±2,0 | 10,2±0,6 | |
| Propionate de testostérone + Produit ex. 13 1 mg/j | 9,9±1,6 | 6,0±0,2 | 61% |
| Propionate de testostérone + Produit ex. 14 1 mg/j | 8,4±0,7 | 4,9±0,3 | 77% |
| Propionate de testostérone + Produit ex. 15 1 mg/j | 7,1±1,0 | 5,3±0,6 | 71% |
| Propionate de testostérone + Produit ex. 16 1 mg/j | 8,5±0,5 | 5,6±0,8 | 67% |
| Propionate de testostérone + Produit ex. 17 1 mg/j | 9,6±1,1 | 8,2±0,9 | 29% |

3) Toxicité aiguë

Cinq souris mâles pesant de 18 à 20 g recoivent en administration par voie intrapéritonéale ou orale des doses décroissantes (1000–600–400 mg/kg) de produit B dissout dans une solution aqueuse de carboxyméthylcellulose (0,25%) et Tween (0,2%). La mortalité est observée sur sept jours de traitement. On détermine le DLO c'est-à-dire la dose la plus élevée pour laquelle on n'observe aucune mortalité.

Résultats:
- Voie intrapéritonéale = DLO = 400 mg/kg
- Voie orale = DLO = 1000 mg/kg.

**Revendications pour les Etats contractants BE, CH, DE, FR, GB, IT, LI, LU, NL, SE**

1. A titre de médicaments et notamment de médicaments antiandrogènes, les produits de formule générale:

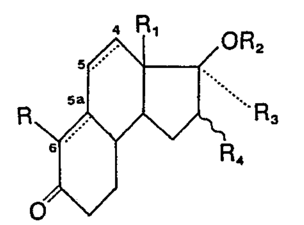

(I)

dans laquelle R représente un radical alkyle linéaire ou ramifié, ayant de 1 à 4 atomes de carbone, $R_1$ représente un radical méthyle ou éthyle, $R_2$ représente un atome d'hydrogène, un radical alkyle, alkényle ou alkynyle, linéaire ou ramifié, ayant au plus 8 atomes de carbone, éventuellement interrompu par un hétéro atome, ou un radical formyle ou acyle ayant de 2 à 18 atomes de carbone, choisi parmi les restes d'un acide aliphatique ou cycloaliphatique saturé ou insaturé, les restes cycloalkylalcanoïques, les radicaux benzoyle ou phénylalcanoyle, les radicaux acyles linéaires comportant un hétéro atome, les radicaux 3-pyridinylcarbonyle, 4-pyridinylcarbonyle, thiazolylcarbonyle, 4,5-dihydrothiazolylcarbonyle, oxazolylcarbonyle ou imidazolylcarbonyle, $R_3$ re-

présente un atome d'hydrogène ou un radical alkyle, alkényle ou alkynyle ayant au plus 4 atomes de carbone, $R_4$ représente un atome d'hydrogène ou un radical alkyle ayant de 1 à 4 atomes de carbone, les traits pointillés indiquent la présence éventuelle d'une seconde liaison entre les carbones 4(5) et 5a(6), le trait ondulé indique que le substituant $R_4$ peut se trouver dans l'une ou l'autre des deux positions possibles α et β.

2. A titre de médicaments, les produits de formule (I) telle que définie à la revendication 1, dans laquelle R représente un radical méthyle ou éthyle et $R_3$ et $R_4$ représentent chacun un atome d'hydrogène.

3. A titre de médicaments, les produits de formule (I), telle que définie à l'une quelconque des revendications 1 ou 2, dans laquelle le trait pointillé en position 4(5) n'indique pas la présence d'une seconde liaison et le trait pointillé en position 5a(6) indique la présence d'une seconde liaison.

4. A titre de médicaments, les produits de formule (I) telle que définie à l'une quelconque des revendications 1 à 3, dans laquelle $R_2$ représente un atome d'hydrogène ou un radical méthoxyméthyle, allyle, acétyle ou butyryle.

5. A titre de médicaments:
la 3a β-méthyl 3β-hydroxy 6-éthyl 1,2,3,3a,4,5,8,9,9a,9b-décahydro 7-H benz (e) inden-7-one,
la 3a β 6-diéthyl 3β-hydroxy 1,2,3,3a,4,5,8,9,9a,9b-décahydro 7-H-benz (e) inden-7-one,
la 3β-(pyridin-3-yl) carbonyloxy 6-éthyl 3aβ-méthyl 1,2,3,3a,4,5,8,9,9a,9b-décahydro 7-H benz (e) inden-7-one.

6. Les compositions pharmaceutiques contenant, à titre de produit actif, au moins un des médicaments tels que définis à l'une quelconque des revendications 1 à 5.

7. Les produits de formule générale (I')

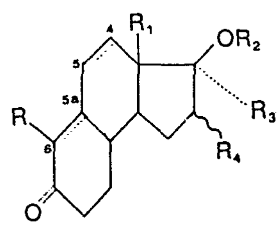

(I')

dans laquelle R, $R_1$, $R_2$, $R_3$, $R_4$, les traits pointillés et ondulés ont les significations indiqués à la revendication 1, à l'exclusion des produits dans lesquels $R_4$ représente un atome d'hydrogène, le trait pointillé en position 4(5) n'indique pas la présence d'une seconde liaison, le trait pointillé en position 5a(6) indique la présence d'une seconde liaison et

a) R représente un radical méthyle et $R_3$ représente un atome d'hydrogène et
   α) $R_2$ représente un atome d'hydrogène, un radical acétyle, benzoyle ou tert-butyle et $R_1$ représente un radical méthyle,
   β) $R_2$ représente un atome d'hydrogène ou un radical tert-butyle et $R_1$ représente un radical éthyle;
b) R représente un radical éthyle ou propyle, $R_3$ représente un atome d'hydrogène, $R_2$ représente un atome d'hydrogène ou un radical benzoyle et $R_1$ représente un radical méthyle;
c) R représente un radical butyle, $R_3$ représente un atome d'hydrogène, $R_2$ représente un atome d'hydrogène et $R_1$ représente un radical méthyle;
d) R représente un radical méthyle, $R_1$ représente un radical méthyle, $R_2$ représente un atome d'hydrogène et $R_3$ représente un radical éthyle ou méthyle;

à l'exclusion également des produits dans lesquels $R_4$ représente un atome d'hydrogène et les traits pointillés n'indiquent pas la présence de secondes liaison et

a) R et $R_1$ représentent chacun un radical méthyle, $R_3$ représente un atome d'hydrogène et $R_2$ représente un atome d'hydrogène ou un radical acétyle ou benzoyle;
b) R et $R_1$ représentent chacun un radical méthyle, $R_2$ représente un atome d'hydrogène et $R_3$ représente un radical éthyle, propargyle ou isobutényle;
c) $R_3$ représente un atome d'hydrogène et
   α) R et $R_1$ représentent chacun un radical méthyle et $R_2$ représente un radical méthoxyméthyle;
   β) R représente un radical éthyle, $R_1$ représente un radical méthyle et $R_2$ représente un atome d'hydrogène;
   γ) R représente un radical méthyle, $R_1$ représente un radical éthyle et $R_2$ représente un atome d'hydrogène;
d) R et $R_1$ représentent chacun un radical méthyle, $R_2$ représente un atome d'hydrogène ou un radical acétyle et $R_3$ représente un radical méthyle.

8. Les produits de formule générale (I') telle que définie à la revendication 7, dans laquelle $R_4$ représente un radical alkyle ayant de 1 à 4 atomes de carbone.

9. Les produits de formule (I') telle que définie à la revendication 7, dans laquelle les traits pointillés indiquent la présence de deux secondes liaisons en position 4(5) et 5a(6).

10. Les produits de formule (I') telle que définie à la revendication 7, dans laquelle le trait pointillé en position 5a(6) indique la présence d'une seconde liaison et les radicaux R et $R_1$ représentent chacun un radical éthyle.

11. La 3aβ 6-diéthyl 3β-hydroxy 1,2,3,3a,4,5,8,9,9a,9b-décahydro 7-H benz (e) inden-7-one;
la 3β-(pyridin-3-yl) carbonyloxy 6-éthyl 3aβ-méthyl 1,2,3,3a,4,5,8,9,9a,9b-décahydro 7-H benz (e) inden-7-one.

12. Procédé de préparation des produits de formule (I') définie à la revendication 7, caractérisé en ce que:

1) Pour préparer les produits de formule ($I'_A$)

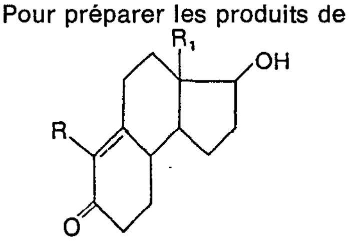

($I'_A$)

dans laquelle R et $R_1$ ont la signification indiquée à la revendication 1, étant entendu que R ne peut pas représenter un radical méthyle, éthyle, n-propyle et n-butyle, lorsque $R_1$ représente un radical méthyle et que R ne peut pas représenter un radical méthyle, lorsque $R_1$ représente un radical éthyle, on fait agir un produit de formule ($II_A$):

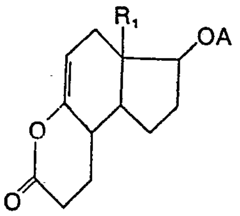

($II_A$)

avec un produit de formule ($III_A$)

$$R\text{–}CH_2\,Mg\,X \qquad (III_A)$$

formules dans lesquelles R et $R_1$ ont les significations précédentes A représente un radical acyle ayant de 2 à 18 atomes de carbone et X représente un atome d'halogène, et saponifie si désiré le produit obtenu;

2) Pour préparer les produits de formule ($I'_B$)

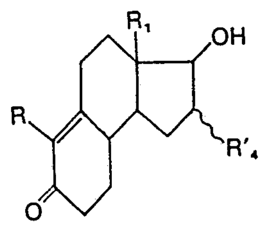

($I'_B$)

dans laquelle R et $R_1$ ont la signification indiquée à la revendication 1 et $R'_4$ représente un radical alkyle ayant de 1 à 4 atomes de carbone, on fait agir un produit de formule ($II_B$)

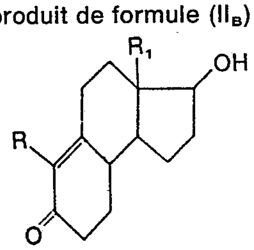

($II_B$)

dans laquelle R et $R_1$ ont les significations indiquées à la revendication 1, avec un réactif de protection du groupement cétonique pour obtenir un produit de formule ($III_B$)

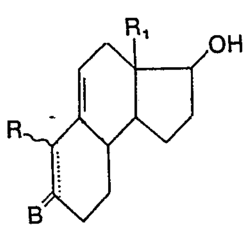

($III_B$)

dans laquelle B représente, soit un groupement cétal, soit un groupement énamine, soit un éther d'énol, le trait pointillé représente une seconde liaison lorsque B est un groupement énamine ou éther d'énol, produit que l'on traite par un agent d'oxydation pour obtenir un produit de formule ($IV_B$):

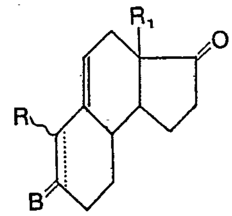

($IV_B$)

que l'on traite, en présence d'une base forte, par un produit de formule $R'_4$ I pour obtenir un produit de formule ($V_B$)

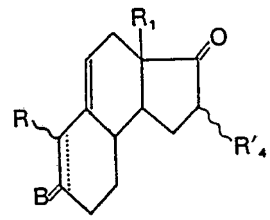

($V_B$)

que l'on soumet à l'action d'un réducteur pour obtenir un produit de formule ($V'_B$):

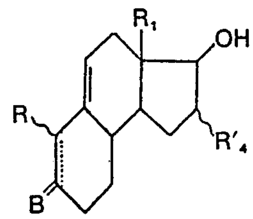

($V'_B$)

que l'on traite de manière à éliminer le radical protecteur de la fonction cétonique pour obtenir le produit de formule ($I'_B$) cherché;

3) Pour préparer les produits de formule ($I'_C$)

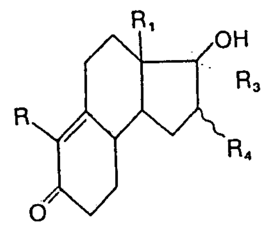

($I'_C$)

dans laquelle R, $R_1$, $R_z$ et $R_4$ ont la signification indiquée à la revendication 1, étant entendu que lorsque $R_4$ représente un atome d'hydrogène, R et $R_1$ ne peuvent pas représenter chacun un radical méthyle lorsque $R_3$ représente un radical éthyle, on fait agir un dérivé de formule $R_3$–D dans laquelle D représente un groupement Mg–Hal, dans lequel Hal représente un atome d'halogène, ou un atome de lithium, sur un dérivé de formule ($II_C$)

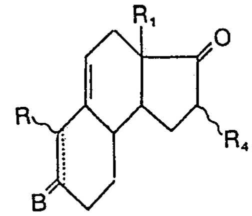

($II_C$)

dans laquelle B, R, $R_1$, $R_4$ et le trait pointillé ont la signification précédente et traite le produit obtenu de manière à éliminer le radical protecteur de la fonction cétonique;

4) Pour préparer les produits de formule ($I'_D$)

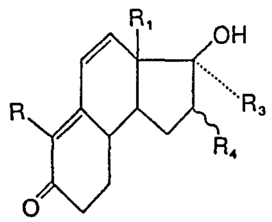

($I'_D$)

dans laquelle R, $R_1$, $R_3$ et $R_4$ ont la signification indiquée à la revendication 1, on fait agir un dérivé réactif d'un groupement protecteur du groupement hydroxyle sur un produit de formule ($II_D$)

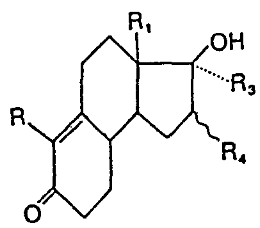

($II_D$)

dans laquelle R, $R_1$, $R_3$ et $R_4$ on la signification indiquée à la revendication 1, pour obtenir un produit de formule ($III_D$)

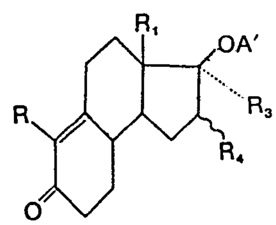

($III_D$)

dans laquelle A' représente un radical protecteur du groupement hydroxyle, que l'on traite par un réactif de bromuration pour obtenir un produit de formule ($IV_D$):

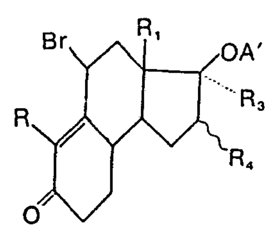

($IV_D$)

que l'on traite d'abord par un agent de débromhydratation puis par un agent permettant d'éliminer le groupement protecteur du radical hydroxyle, pour obtenir le produit $I'_D$ recherché;

5) Pour préparer les produits de formule ($I'_E$)

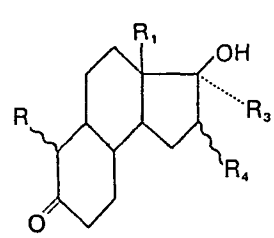

($I'_E$)

dans laquelle R, $R_1$, $R_3$ et $R_4$ ont la signification indiquée à la revendication 1, étant entendu que lorsque $R_4$ représente un atome d'hydrogène,

a) lorsque $R_3$ représente un atome d'hydrogène, un radical méthyle, éthyle, propargyle ou isobutényle, R et $R_1$ ne peuvent pas représenter chacun un radical méthyle,

b) lorsque $R_3$ représente un atome d'hydrogène, R ne peut pas représenter un radical méthyle lorsque $R_1$ représente un radical éthyle et inversément, R ne peut pas représenter un radical éthyle lorsque $R_1$ représente un radical méthyle, on fait agir un réactif d'hydrogénation sur un produit de formule ($II'_E$):

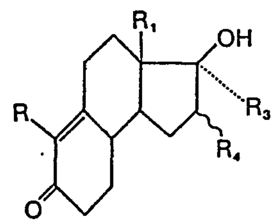

($II'_E$)

dans laquelle R, $R_1$, $R_3$ et $R_4$ ont la signification précédente;

6) Pour préparer les produits de formule ($I'_F$):

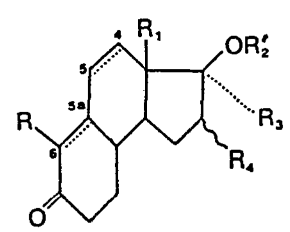

($I'_F$)

dans laquelle R, $R_1$, $R_3$, $R_4$ et les traits pointillés ont les significations indiquées à la revendication 1 et $R'_2$ a les significations de $R_2$ à l'exclusion de la valeur hydrogène, étant entendu

– que sont exclus les produits dans lesquels R représente un atome d'hydrogène, le trait pointillé en position 4(5) n'indique pas la présence d'une seconde liaison et le trait pointillé en position 5a(6) indique la présence d'une seconde liaison et

a) R représente un radical méthyle et $R_3$ représente un atome d'hydrogène et

α) $R_1$ représente un radical méthyle et $R'_2$ représente un radical acétyle, benzoyle ou tert-butyle,

β) $R_1$ représente un radical éthyle et $R'_2$ représente un radical tert-butyle,

b) R représente un radical éthyle ou propyle, $R_1$ représente un radical méthyle, $R'_2$ représente un radical benzoyle et $R_3$ représente un atome d'hydrogène,

– que sont exclus également les produits dans lesquels $R_4$ représente un atome d'hydrogène, les traits pointillés n'indiquent pas la présence de secondes liaisons et

a) R et $R_1$ représentent chacun un radical méthyle, $R_3$ représente un atome d'hydrogène et $R'_2$ représente un radical acétyle ou benzoyle,

b) $R_3$ représente un atome d'hydrogène, R et $R_1$ représentent chacun un radical méthyle et R'2 représente un radical méthoxyméthyle,

c) R, $R_1$ et $R_3$ representent chacun un radical méthyle et $R'_2$ représente un radical acétyle,

soit l'on fait agir un dérivé fonctionnel de formule $R'_2$–E dans laquelle E représente le reste d'un groupement fonctionnel choisi parmi les halogénures d'alkyle, alkényle et alkynyle ou les halogénures ou anhydrides mixtes ou symétriques d'acyles et $R'_2$ a la signification précédente, sur un produit de formule ($II_F$):

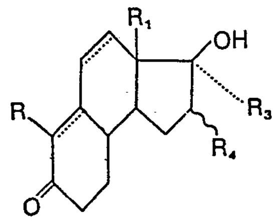

($II_F$)

dans laquelle R, $R_1$, $R_3$ et $R_4$ ont la signification précédente, étant entendu que les valeurs des substituants R, $R_1$, $R'_2$, $R_3$, $R_4$ ainsi que les traits pointillés sont soumis aux restrictions précédentes, pour obtenir un produit de formule ($I'_F$) recherché, soit pour préparer les produits de formule ($I'_F$) dans laquelle le trait pointillé en position 4(5) ne représente pas une seconde liaison, on fait agir un produit de formule ($II'_F$):

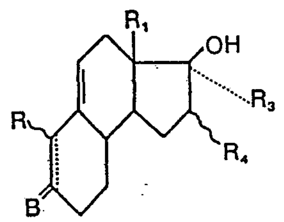

($II'_F$)

dans laquelle B a la signification précédente et R, $R_1$, $R_3$ et $R_4$ sont soumis aux restrictions précédentes, avec un dérivé fonctionnel de formule

$R'_2$–E

dans laquelle $R'_2$ et E ont la signification précédente, pour obtenir un produit de formule ($III'_F$):

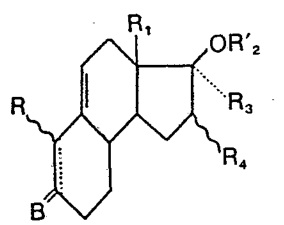

($III'_F$)

que l'on traite de manière à éliminer le radical protecteur de la fonction cétonique, pour obtenir le produit de formule ($I'_F$) dans laquelle le trait pointillé en position 4(5) ne représente pas une seconde liaison.

13. A titre de médicament la 3aβ-méthyl 3β-acétoxy 6-éthyl 1,2,3,3a,4,5,8,9,9a,9b-décahydro 7-H benz (e) inden-7-one.

14. Les compositions pharmaceutiques contenant, á titre de produit actif le médicament selon la revendication 13.

15. La 3aβ-méthyl 3β-acétoxy 6-éthyl 1,2,3,3a,4,5,8,9,9a,9b-décahydro 7-H benz (e) inden-7-one.

**Revendication pour l'Etat contractant AT**

1. Procédé de préparation des produits de formule générale (I'):

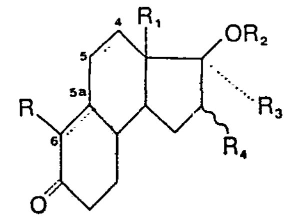

(I')

dans laquelle R représente un radical alkyle linéaire ou ramifié, ayant de 1 à 4 atomes de carbone, $R_1$ représente un radical méthyle ou éthyle, $R_2$ représente un atome d'hydrogène, un radical alkyle, alkényle ou alkynyle, linéaire ou ramifié, ayant au plus 8 atomes de carbone, éventuellement interrompu par un hétéro atome, ou un radical formyle ou acyle ayant de 2 à 18 atomes de carbone, choisi parmi les restes d'un acide aliphatique ou cycloaliphatique saturé ou insaturé, les restes cycloalkylalcanoïques, les radicaux benzoyle ou phénylalcanoyle, les radicaux acyles linéaires comportant un hétéro atome, les radicaux 3-pyridinylcarbonyle, 4-pyridinylcarbonyle, thiazolylcarbonyle, 4,5-dihydrothiazolylcarbonyle, oxazolylcarbonyle ou imidazolylcarbonyle, $R_3$ représente un atome d'hydrogène ou un radical alkyle, alkényle ou alkynyle ayant au plus 4 atomes de carbone, $R_4$ représente un atome d'hydrogène ou un radical alkyle ayant de 1 à 4 atomes de carbone, les traits pointillés indiquent la présence éventuelle d'une seconde liaison entre les carbones 4(5) et 5a(6), le trait ondulé indique que le substituant $R_4$ peut se trouver dans l'une ou l'autre des deux positions possibles α ou β, à l'exclusion des produits dans lesquels $R_4$ représente un atome d'hydrogène, le trait pointillé en position 4(5) n'indique pas la présence d'une seconde liaison, le trait pointillé en position 5a(6) indique la présence d'une seconde liaison et

a) R représente un radical méthyle et $R_3$ représente un atome d'hydrogène et
   α) $R_2$ représente un atome d'hydrogène, un radical acétyle, benzoyle ou tert-butyle et $R_1$ représente un radical méthyle,
   β) $R_2$ représente un atome d'hydrogène ou un radical tert-butyle et $R_1$ représente un radical éthyle;
b) R représente un radical éthyle ou propyle, $R_3$ représente un atome d'hydrogène, $R_2$ représente un atome d'hydrogène ou un radical benzoyle et $R_1$ représente un radical méthyle;
c) R représente un radical butyle, $R_3$ représente un atome d'hydrogène, $R_2$ représente un atome d'hydrogène et $R_1$ représente un radical méthyle;
d) R représente un radical méthyle, $R_1$ représente un radical méthyle, $R_2$ représente un atome d'hydrogène et $R_3$ représente un radical éthyle;

à l'exclusion également des produits dans lesquels $R_4$ représente un atome d'hydrogène et les

traits pointillés n'indiquent pas la présence de secondes liaisons et

a) R et $R_1$ représentent chacun un radical méthyle, $R_3$ représente un atome d'hydrogène et $R_2$ représente un atome d'hydrogène ou un radical acétyle ou benzoyle;

b) R et $R_1$ représentent chacun un radical méthyle, $R_2$ représente un atome d'hydrogène et $R_3$ représente un radical éthyle, propargyle ou isobuténylе;

c) $R_3$ représente un atome d'hydrogène et

α) R et $R_1$ représentent chacun un radical méthyle et $R_2$ représente un radical méthoxyméthyle;

β) R représente un radical éthyle, $R_1$ représente un radical méthyle et $R_2$ représente un atome d'hydrogène;

γ) R représente un radical méthyle, $R_1$ représente un radical éthyle et $R_2$ représente un atome d'hydrogène;

d) R et $R_1$ représentent chacun un radical méthyle, $R_2$ représente un atome d'hydrogène ou un radical acétyle et $R_3$ représente un radical méthyle, caractérisé en ce que:

1) Pour préparer les produits de formule ($I'_A$)

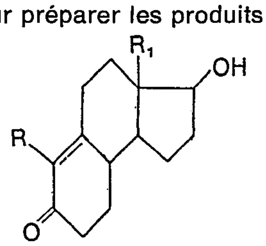

($I'_A$)

dans laquelle R et $R_1$ ont la signification indiquée précédemment, étant entendu que R ne peut pas représenter un radical méthyle, éthyle, n-propyle et n-butyle, lorsque R1 représente un radical méthyle et que R ne peut pas représenter un radical méthyle, lorsque $R_1$ représente un radical éthyle, on fait agir un produit de formule ($II_A$):

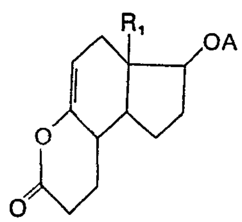

($II_A$)

avec un produit de formule ($III_A$)

$$R\text{–}CH_2\,Mg\,X \qquad (III_A)$$

formules dans lesquelles R et $R_1$ ont les significations précédentes, A représente un radical acyle ayant de 2 à 18 atomes de carbone et X représente un atome d'alogène, et saponifie si désiré le produit obtenu;

2) Pour préparer les produits de formule ($I'_B$)

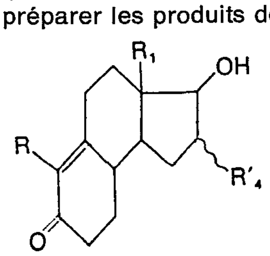

($I'_B$)

dans laquelle R et $R_1$ ont la signification indiquée précédemment et $R'_4$ représente un radical alkyle ayant de 1 à 4 atomes de carbone, on fait agir un produit de formule ($II_B$)

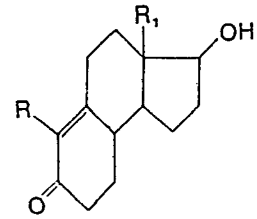

($II_B$)

dans laquelle R et $R_1$ ont les significations indiquées précédemment, avec un réactif de protection du groupement cétonique pour obtenir un produit de formule ($III_B$)

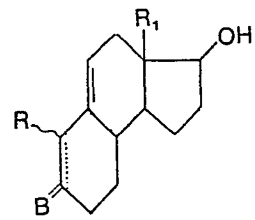

($III_B$)

dans laquelle B représente, soit un groupement cétal, soit un groupement énamine, soit un éther d'énol, le trait pointillé représente une seconde liaison lorsque B est un groupement énamine ou éther d'énol, produit que l'on traite par un agent d'oxydation pour obtenir un produit de formule ($IV_B$):

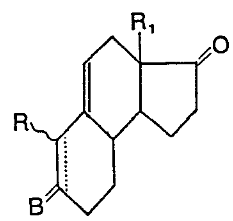

($IV_B$)

que l'on traite, en présence d'une base forte, par un produit de formule $R'_4$ I pour obtenir en produit de formule ($V_B$)

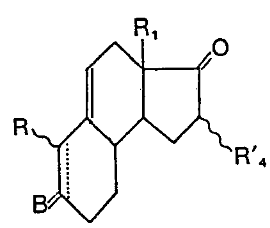

($V_B$)

que l'on soumet à l'action d'un réducteur pour obtenir un produit de formule ($V'_B$):

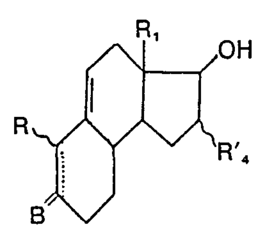

($V'_B$)

que l'on traite de manière à éliminer le radical protecteur de la fonction cétonique pour obtenir le produit de formule ($I'_B$) cherché;

3) Pour préparer les produits formule ($I'_C$)

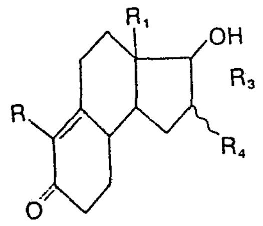

($I'_C$)

dans laquelle R, $R_1$, $R_3$ et $R_4$ ont la signification indiquée précédemment, étant entendu que lorsque $R_4$ représente un atome d'hydrogène, R et $R_1$ ne peuvent pas représenter chacun un radical méthyle lorsque $R_3$ représente un radical éthyle, on fait agir un dérivé de formule $R_3$–D dans laquelle D représente un groupement Mg–Hal, dans lequel Hal représente un atome d'halogène, ou un atome de lithium, sur un dérivé de formule ($II_C$)

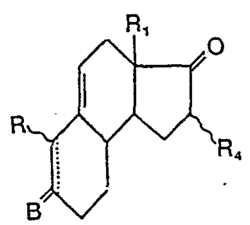

($II_C$)

dans laquelle B, R, $R_1$, $R_4$ et le trait pointillé ont la signification précédente et traite le produit obtenu de manière à éliminer le radical protecteur de la fonction cétonique;

4) Pour préparer les produits de formule ($I'_D$)

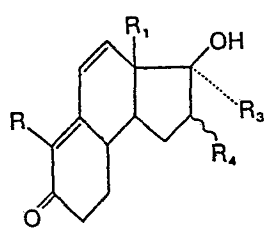

($I'_D$)

dans laquelle R, $R_1$, $R_3$ et $R_4$ ont la signification indiquée précédemment, on fait agir un dérivé réactif d'un groupement protecteur du groupement hydroxyle sur un produit de formule ($II_D$)

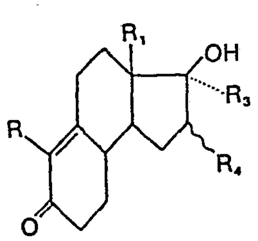

($II_D$)

dans laquelle R, $R_1$, $R_3$ et $R_4$ ont la signification indiquée précedemment, pour obtenir un produit de formule ($III_D$)

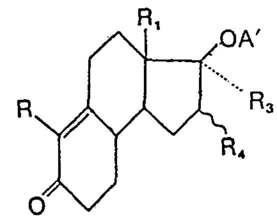

($III_D$)

dans laquelle A' représente un radical protecteur du groupement hydroxyle, que l'on traite par un réactif de bromuration pour obtenir un produit de formule ($IV_D$):

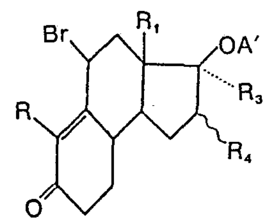

($IV_D$)

que l'on traite d'abord par un agent de débromhydratation puis par un agent permettant d'éliminer le groupement protecteur du radical hydroxyle, pour obtenir le produit $I'_D$ recherché;

5) Pour préparer les produits de formule ($I'_E$)

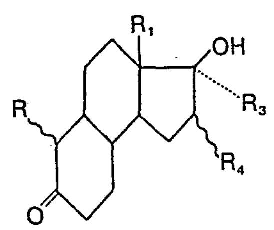

($I'_E$)

dans laquelle R, $R_1$, $R_3$ et $R_4$ ont la signification indiquée précédemment, étant entendu que lorsque $R_4$ représente un atome d'hydrogène,

a) lorsque $R_3$ représente un atome d'hydrogène, un radical méthyle, éthyle, propargyle ou isobutényle, R et $R_1$ ne peuvent pas représenter chacun un radical méthyle,

b) lorsque $R_3$ représente un atome d'hydrogène, R ne peut pas représenter un radical méthyle lorsque $R_1$ représente un radical éthyle et inversement, R ne peut pas représenter un radical éthyle lorsque $R_1$ représente un radical méthyle, on fait agir un réactif d'hydrogénation sur un produit de formule ($II'_E$):

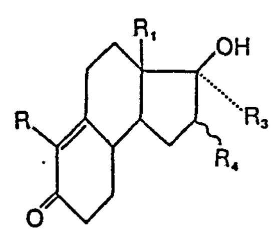

($II'_E$)

dans laquelle R, $R_1$, $R_3$ et $T_4$ ont la signification précédente;

6) Pour préparer les produits de formule ($I'_F$):

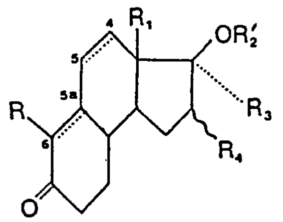

($I'_F$)

dans laquelle R, $R_1$, $R_3$ $R_4$ et les traits pointillés ont les significations indiquées précédemment et $R'_2$ a les significations de $R_2$ à l'exclusion de la valeur hydrogène, étant entendu que sont exclus les produits dans lesquels $R_4$ représente un atome d'hydrogène, le trait pointillé en position 4(5) n'indique pas la présence d'une seconde liaison et le trait pointillé en position 5a(6) indique la présence d'une seconde liaison et

a) R représente un radical méthyle et $R_3$ représente un atome d'hydrogène et
   α) $R_1$ représente un radical méthyle et $R_2$ représente un radical acétyle, benzoyle ou tert-butyle,
   β) $R_1$ représente un radical éthyle et $R_2$ représente un radical tert-butyle,
b) R représente un radical éthyle ou propyle, $R_1$ représente un radical méthyle, $R_2$ représente un radical benzoyle et $R_3$ représente un atome d'hydrogène,

que sont exclus également les produits dans lesquels $R_4$ représente un atome d'hydrogène, les traits pointillés n'indiquent pas la présence de secondes liaisons et

a) R et $R_1$ représentent chacun un radical méthyle, $R_3$ représente un atome d'hydrogène et $R'_2$ représente un radical acétyle ou benzoyle,
b) $R_3$ représente un atome d'hydrogène, R et $R_1$ représentent chacun un radical méthyle et $R'_2$ représente un radical méthoxyméthyle,
c) R, $R_1$ et $R_3$ représentent chacun un radical méthyle et $R'_2$ représente un radical acétyle,

soit l'on fait agir un dérivé fonctionnel de formule $R'_2$–E dans laquelle E représente le reste d'un groupement fonctionnel choisi parmi les halogénures d'alkyle, alkényle ou alkynyle ou les halogénures ou anhydrides mixtes ou symétriques d'acyles et $R'_2$ a la signification précédente, sur un produit de formule ($II_F$):

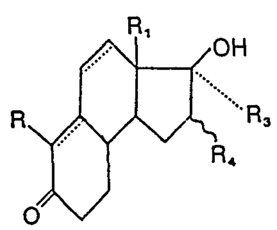

($II_F$)

dans laquelle R, $R_1$, $R_3$ et $R_4$ ont la signification précédente, étant entendu que les valeurs des substituants R, $R_1$, $R'_2$, $R_3$, $R_4$ ainsi que les traits pointillés sont soumis aux restrictions précédentes, pour obtenir un produit de formule ($I'_F$) recherché, soit pour préparer les produits de formule ($I'_F$) dans laquelle le trait pointillé en position 4(5) ne représente pas une seconde liaison, on fait agir un produit de formule ($II'_F$):

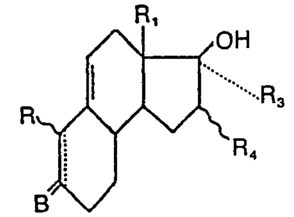

($II'_F$)

dans laquelle B a la signification précédente et R, $R_1$, $R_3$ et $R_4$ sont soumis aux restrictions précédentes, avec un dérivé fonctionnel de formule

$$R'_2\text{–}E$$

dans laquelle $R'_2$ et E ont la signification précédente, pour obtenir un produit de formule ($III'_F$):

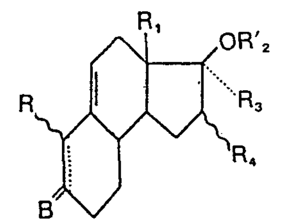

($III'_F$)

que l'on traite de manière à éliminer le radical protecteur de la fonction cétonique, pour obtenir le produit de formule ($I'_F$) dans laquelle le trait pointillé en position 4(5) ne représente pas une seconde liaison.

**Claims for the contracting States BE, CH, DE, FR, GB, IT, LI, LU, NL, SE**

1. As medicaments and in particular as antiandrogene medicaments, the products with the general formula:

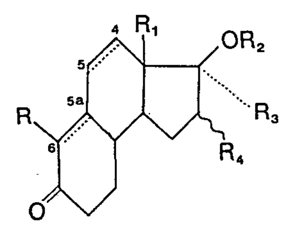

(I)

in which R represents a linear or branched alkyl radical, having from 1 to 4 carbon atoms, $R_1$ represents a methyl or ethyl radical, $R_2$ represents a hydrogen atom, a linear or branched alkyl, alkenyl or alkynyl radical, having at the most 8 carbon atoms, possibly interrupted by a hetero atom, or a formyl or acyl radical having from 2 to 18 carbon atoms, chosen from among the residues of an aliphatic or cycloaliphatic acid saturated or unsaturated, the cycloalkylalkanoic residues, the benzoyl or phenylalkanoyl radicals, the linear acyl radicals including a hetero atom, the 3-pyridinylcarbonyl, 4-pyridinylcarbonyl, thiazolylcarbonyl, 4,5-dihydrothiazolylcarbonyl, oxazolylcarbonyl or imidazolylcarbonyl radicals, $R_3$ represents a hy-

drogen atom or an alkyl, alkenyl or alkynyl radical having at the most 4 carbon atoms, $R_4$ represents a hydrogen atom or an alkyl radical having from 1 to 4 carbon atoms, the dotted lines indicate the possible presence of a second bond between the carbons 4(5) and 5a(6), the wavy line indicates that the substituent $R_4$ may be found in one of the two possible positions α or β.

2. As medicaments, the products with the formula (I) as defined in Claim 1, in which R represents a methyl or ethyl radical and $R_3$ and $R_4$ each represent a hydrogen atom.

3. As medicaments, products with the formula (I), as defined in either of the Claims 1 or 2, in which the dotted line at position 4(5) does not indicate the presence of a second bond and the dotted line at position 5a(6) indicates the presence of a second bond.

4. As medicaments, the products with the formula (I) as defined in any one of the Claims 1 to 3, in which $R_2$ represents an hydrogen atom or a methoxymethyl, allyl, acetyl or butyryl atom.

5. As medicaments:

3a-β-methyl-3β-hydroxy-6-ethyl-1,2,3,3a,4,5,-8,9,9a,9b-decahydro-7-H-benz-(e)-inden-7-one,

3α-β-6-diethyl-3β-hydroxy-1,2,3,3a,4,5,8,9,9a,9b-decahydro-7-H-benz-(e)-inden-7-one,

3β-(pyridin-3-yl)carbonyloxy-6-ethyl-3αβ-methyl-1,2,3,3a,4,5,8,9,9a,9b-decahydro-7-H-benz-(e)-inden-7-one.

6. Pharmaceutical compositions, containing as active product, at least one of the medicaments as defined in any one of the Claims 1 to 5.

7. The products with the general formula (I')

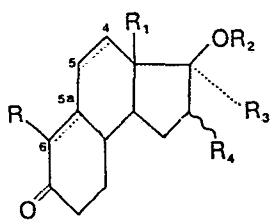

(I')

in which R, $R_1$, $R_2$, $R_3$, $R_4$, the dotted lines and the wavy line have the significances indicated in Claim 1, with the exception of the products in which $R_4$ represents a hydrogen atom, the dotted line at position 4(5) does not indicate the presence of a second bond, the dotted line at position 5a(6) indicates the presence of a second bond and

a) R represents a methyl redical and $R_3$ represents a hydrogen atom and
   α) $R_2$ represents a hydrogen atom, an acetyl, benzoyl or tert-butyl radical and $R_1$ represents a methyl radical,
   β) $R_2$ represents a hydrogen atom or a tert-butyl radical and $R_1$ represents an ethyl radical;
b) R represents an ethyl or propyl radical, $R_3$ represents a hydrogen atom, $R_2$ represents a hydrogen atom or a benzoyl radical and $R_1$ represents a methyl radical;
c) R represents a butyl radical, $R_3$ represents a hydrogen atom, $R_2$ represents a hydrogen atom and $R_1$ represents a methyl radical;
d) R represents a methyl radical, $R_1$ represents a methyl radical, $R_2$ represents a hydrogen atom and $R_3$ represents an ethyl or methyl radical;

with the exception also of the products in which $R_4$ represents a hydrogen atom and the dotted lines do not indicate the presence of second bonds and

a) R and $R_1$ each represent a methyl radical, $R_3$ represents a hydrogen atom and $R_2$ represents a hydrogen atom or an acetyl or benzoyl radical;
b) R and $R_1$ each represent a methyl radical, $R_2$ represents a hydrogen atom and $R_3$ represents an ethyl, propargyl or isobutenyl radical;
c) $R_3$ represents a hydrogen atom and
   α) R and $R_1$ each represent a methyl radical and $R_2$ represents a methoxymethyl radical;
   β) R represents an ethyl radical, $R_1$ represents a methyl radical and $R_2$ represents a hydrogen atom;
   γ) R represents a methyl radical, $R_1$ represents an ethyl radical and $R_2$ represents a hydrogen atom;
d) R and $R_1$ each represent a methyl radical, $R_2$ represents a hydrogen atom or an acetyl radical and $R_3$ represents a methyl radical.

8. Products with the general formula (I') as defined in Claim 7, in which $R_4$ represents an alkyl radical having from 1 to 4 carbon atoms.

9. Products with the formula (I') as defined in Claim 7, in which the dotted lines indicate the presence of two second bonds at the position 4(5) and 5a(6).

10. Products with the formula (I') as defined in Claim 7, in which the dotted line at position 5a(6) indicates the presence of a second bond and the radicals R and $R_1$ each represent an ethyl radical.

11. 3aβ-6-diethyl-3β-hydroxy-1,2,3,3a,4,5,8,9,9a,9b-decahydro-7-H-benz-(e)-inden-7-one;

3β-(pyridin-3-yl)carbonyloxy-6-ethyl-3aβ-methyl-1,2,3,3a,4,5,8,9,9a,9b-decahydro-7-H-benz-(e)-inden-7-one.

12. Preparation process of products with the formula (I') defined in Claim 7, characterized in that:

1) To prepare the products with the formula ($I'_A$)

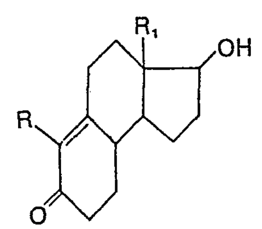

($I'_A$)

in which R and $R_1$ have the significance mentioned in Claim 1, it being understood that R can not represent a methyl, ethyl, n-propyl and n-butyl radical, when $R_1$ represents a methyl radical and that R can not represent a methyl radical, when $R_1$ represents an ethyl radical, a product with the formula ($II_A$):

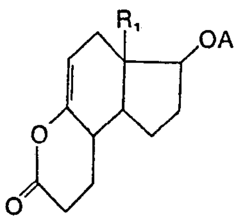

$(II_A)$

is made to react with a product with the formula $(III_A)$

$$R{-}CH_2\ Mg\ X \qquad (III_A)$$

in which formulae R and $R_1$ have the previous significances, A represents an acyl radical having from 2 to 18 carbon atoms and X represents a halogen atom, and the product obtained is saponified if desired;

2) To prepare the products with the formula $(I'_B)$

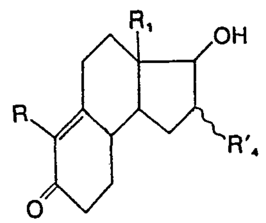

$(I'_B)$

in which R and $R_1$ have the significance mentioned in Claim 1 and $R'_4$ represents an alkyl radical having from 1 to 4 carbon atoms, a product with the formula $(II_B)$

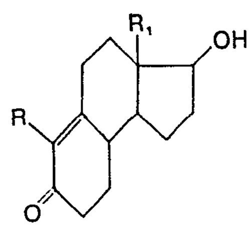

$(II_B)$

in which R and $R_1$ have the significances mentioned in Claim 1, is made to react with a protection reagent of the ketone group so as to obtain a product with the formula $(III_B)$

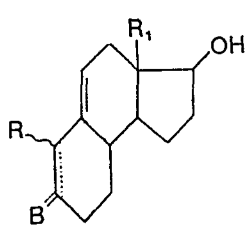

$(III_B)$

in which B represents, either a ketal group, or an enamine group or an enol ether, the dotted line represents a second bond when B is an enamine group or an enol ether, this product is treated with an oxydation agent so as to obtain a product with the formula $(IV_B)$:

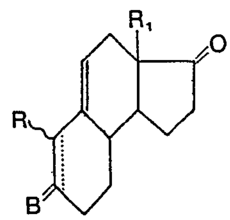

$(IV_B)$

which is treated, in the presence of a strong base with a product with the formula $R'_4I$ so as to obtain a product with the formula $(V_B)$

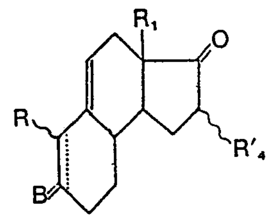

$(V_B)$

which is submitted to the action of a reducing agent so as to obtain a product with the formula $(V'_B)$:

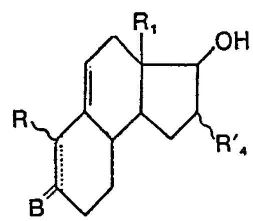

$(V'_B)$

which is treated so as to eliminate the protector radical of the ketone function so as to obtain the product with the formula $(I'_B)$ sought;

3) To prepare the products with the formula $(I'_C)$

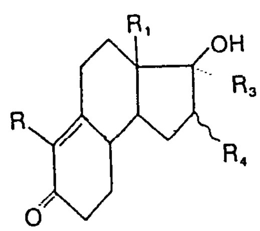

$(I'_C)$

in which R, $R_1$, $R_3$ and $R_4$ have the significance indicated in Claim 1, it being understood that when $R_4$ represents a hydrogen atom, R and $R_1$ can not each represent a methyl radical when $R_3$ represents an ethyl radical, a derivative with the formula $R_3$–D in which D represents a group Mg–Hal, in which Hal represents a halogen atom, or a lithium atom, is made to react with a derivative with the formula $(II_C)$

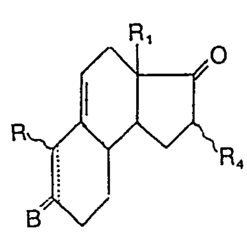

$(II_C)$

in which B, R, $R_1$, $R_4$ and the dotted line have the previous significance and the product obtained is treated so as to eliminate the protector radical of the ketone function;

4) To prepare the products with the formula ($I'_D$)

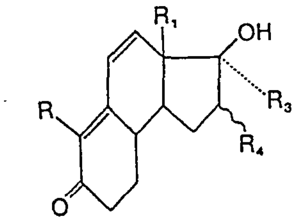

($I'_D$)

in which R, $R_1$, $R_3$ and $R_4$ have the significance indicated in Claim 1, a reactive derivative of a protector group of the hydroxyl group is made to react with a product with the formula ($II_D$)

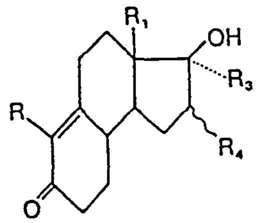

($II_D$)

in which R, $R_1$, $R_3$ and $R_4$ have the significance indicated in Claim 1, so as to obtain a product with the formula ($III_D$)

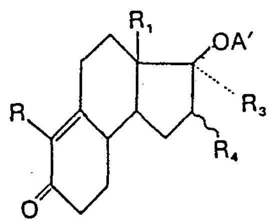

($III_D$)

in which A′ represents a protector radical of the hydroxyl group, which is treated with a bromiding reagent so as to obtain a product with the formula ($IV_D$):

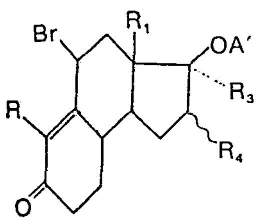

($IV_D$)

which is treated first with a debromhydration agent then by an agent which allows the protector group of the hydroxyl radical to be eliminated, so as to obtain the sought product $I'_D$;

5) To prepare the products with the formula ($I'_E$)

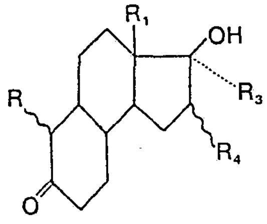

($I'_E$)

in which R, $R_1$, $R_3$ and $R_4$ have the significance indicated in Claim 1, it being understood that when $R_4$ represents a hydrogen atom,

a) when $R_3$ represents a hydrogen atom, a methyl, ethyl, propargyl or isobutenyl radical, R and $R_1$ each can not represent a methyl radical,
b) when $R_3$ represents a hydrogen atom, R can not represent a methyl radical when $R_1$ represents an ethyl radical and conversely, R can not represent an ethyl radical when $R_1$ represents a methyl radical, a hydrogenation reagent is made to react with a product with the formula ($II'_E$):

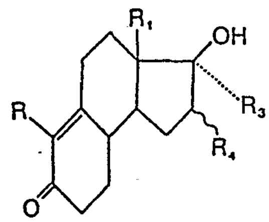

($II'_E$)

in which R, $R_1$, $R_3$ and $R_4$ have the previous significance;

6) To prepare the products with the formula ($I'_F$):

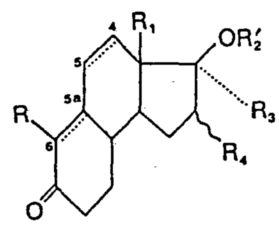

($I'_F$)

in which R, $R^1$, $R_3$ $R_4$ and the dotted lines have the significances indicated in Claim 1 and $R'_2$ has the significances of $R_2$ with the exception of the hydrogen value, it being understood

– that the products are excluded in which $R_4$ represents a hydrogen atom, the dotted line at position 4(5) does not indicate the presence of a second bond and the dotted line at position 5a(6) indicates the presence of a second bond and

a) R represents a methyl radical and $R_3$ represents a hydrogen atom and
   α) $R_1$ represents a methyl radical and $R'_2$ represents a acetyl, benzoyl or tert-butyl radical,
   β) $R_1$ represents an ethyl radical and $R'_2$ represents a tert-butyl radical,
b) R represents an ethyl or propyl radical, $R_1$ represents a methyl radical, $R'_2$ represents a benzoyl radical and $R_3$ represents a hydrogen atom,

– that the products are also excluded in which $R_4$ represents a hydrogen atom, the dotted lines do not indicate the presence of second bonds and

a) R and $R_1$ each represent a methyl radical, $R_3$ represents a hydrogen atom and $R'_2$ represents an acetyl or benzoyl radical,
b) $R_3$ represents a hydrogen atom, R and $R_1$ each represent a methyl radical and $R'_2$ represents a methoxymethyl radical,

c) R, $R_1$ and $R_3$ each represent a methyl radical and $R'_2$ represents a acetyl radical,

either a functional derivative with the formula $R'_2$–E in which E represents the residue of a functional group chosen from among the alkyl, alkenyl or alkynyl halides, or the halides or mixed or symmetric anhydrides of acyls and $R'_2$ has the previous significance, is made to react with a product with the formula ($II_F$):

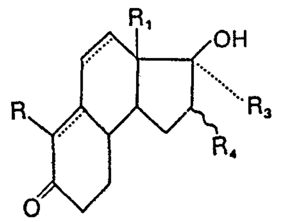

($II_F$)

in which R, $R_1$, $R_3$ and $R_4$ have the previous significance, it being understood that the values of the substituents R, $R_1$, $R'_2$, $R_3$, $R_4$ as well as the dotted lines are submitted to the previous restrictions, so as to obtain a product with the formula ($I'_F$) sought, or to prepare the products with the formula ($I'_F$) in which the dotted line at position 4(5) does not represent a second bond, a product with the formula ($II'_F$)

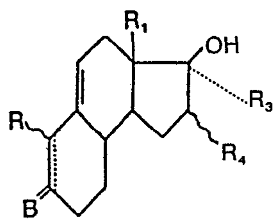

($II'_F$)

in which B has the previous significance and R, $R_1$, $R_3$ and $R_4$ are submitted to the previous restrictions, is made to react with a functional derivative with the formula

$R'_2$–E

in which $R'_2$ and E have the previous significance, so as to obtain a product with the formula ($III'_F$):

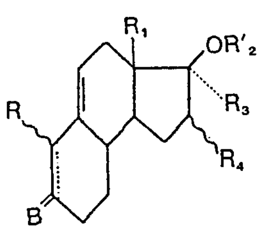

($III'_F$)

which is treated so as to eliminate the protector radical of the ketone function, so as to obtain the product with the formula ($I'_F$) in which the dotted line at position 4(5) does not represent a second bond.

13. As medicament 3aβ-methyl-3β-acetoxy-6-ethyl-1,2,3,3a,4,5,8,9,9a,9b-decahydro-7-H-benz-(e)-inden-7-one.

14. Pharmaceutical compositions containing, as active product the medicament according to Claim 13.

15. 3aβ-methyl-3β-acetoxy-6-ethyl-1,2,3,3a,4,5,8,9,9a,9b-decahydro-7-H-benz-(e)-inden-7-one.

**Claim for the contracting State AT**

1. Preparation process of products with the general formula (I′):

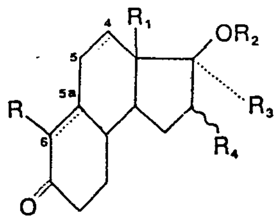

(I′)

in which R represents a linear or branched alkyl radical, having from 1 to 4 carbon atoms, $R_1$ represents a methyl or ethyl radical, $R_2$ represents a hydrogen atom, a linear or branched alkyl, alkenyl or alkynyl radical, having at the most 8 carbon atoms, possibly interrupted by a hetero atom, or an acyl or formyl radical having from 2 to 18 carbon atoms, chosen from among the residues of a saturated or unsaturated aliphatic or cycloaliphatic acid, the cycloalkylalkanoic residues, the benzoyl or phenylalkanoyl radicals, the linear acyl radicals including a hetero atom, the 3-pyridinylcarbonyl, 4-pyridinylcarbonyl, thiazolylcarbonyl, 4,5-dihydrothiazolylcarbonyl, oxazolylcarbonyl or imidazolylcarbonyl radicals, $R_3$ represents a hydrogen atom or an alkyl, alkenyl or alkynyl radical having at the most 4 carbon atoms, $R_4$ represents a hydrogen atom or an alkyl radical having from 1 to 4 carbon atoms, the dotted lines indicate the possible presence of a second bond between the carbons (4(5) and 5a(6), the wavy line indicates that the substituent $R_4$ may be found in one of the two possible positions α or β, with the exception of the products in which $R_4$ represents a hydrogen atom, the dotted line at position 4(5) does not indicate the presence of a second bond, the dotted line at position 5a(6) indicates the presence of a second bond and

a) R represents a methyl radical and $R_3$ represents a hydrogen atom and
   α) $R_2$ represents a hydrogen atom, an acetyl, benzoyl or tert-butyl radical and $R_1$ represents a methyl radical,
   β) $R_2$ represents a hydrogen atom or a tert-butyl radical and $R_1$ represents an ethyl radical;
b) R represents an ethyl or propyl radical, $R_3$ represents a hydrogen atom, $R_2$ represents a hydrogen atom or a benzoyl radical and $R_1$ represents a methyl radical;
c) R represents a butyl radical, $R_3$ represents a hydrogen atom, $R_2$ represents a hydrogen atom and $R_1$ represents a methyl radical;
d) R represents a methyl radical, $R_1$ represents a methyl radical, $R_2$ represents a hydrogen atom and $R_3$ represents an ethyl radical;

with the exception also of the products in which $R_4$ represents a hydrogen atom and the dotted lines do not indicate the presence of second bonds and

a) R and $R_1$ each represent a methyl radical, $R_3$ represents a hydrogen atom and $R_2$ represents a hydrogen atom or an acetyl or benzoyl radical;

b) R and $R_1$ each represent a methyl radical, $R_2$ represents a hydrogen atom and $R_3$ represents an ethyl, propargyl or isobutenyl radical;

c) $R_3$ represents a hydrogen atom and
 α) R and $R_1$ each represent a methyl radical and $R_2$ represents a methoxymethyl radical;
 β) R represents an ethyl radical, $R_1$ represents a methyl radical and $R_2$ represents a hydrogen atom;
 γ) R represents a methyl radical, $R_1$ represents an ethyl radical and $R_2$ represents a hydrogen atom;

d) R and $R_1$ each represent a methyl radical, $R_2$ represents a hydrogen atom or an acetyl radical and $R_3$ represents a methyl radical, characterized in that:

1) To prepare the products with the formula ($I'_A$)

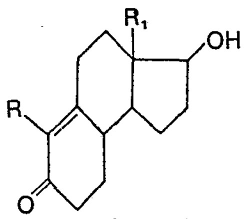

($I'_A$)

in which R and $R_1$ have the previously indicated significance, it being understood that R can not represent a methyl, ethyl, n-propyl and n-butyl radical, when $R_1$ represents a methyl radical and that R can not represent a methyl radical, when $R_1$ represents an ethyl radical, a product with the formula ($II_A$):

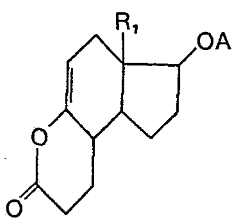

($II_A$)

is made to react with a product with the formula ($III_A$)

$$R\text{–}CH_2\,Mg\,X \qquad (III_A)$$

in which formulae R and $R_1$ have the previous significances, A represents an acyl radical having from 1 to 18 carbon atoms and X represents a halogen atom, and the product obtained is saponified if desired;

2) To prepare the products with the formula ($I'_B$)

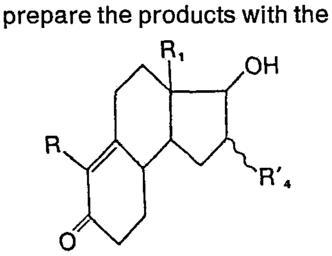

($I'_B$)

in which R and $R_1$ have the significance mentioned previously and $R'_4$ represents an alkyl radical having from 1 to 4 carbon atoms, a product with the formula ($II_B$)

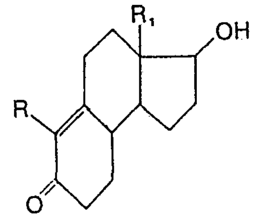

($II_B$)

in which R and $R_1$ have the significances mentioned previously, is made to react with a protection reagent of the ketone group so as to obtain a product with the formula ($III_B$)

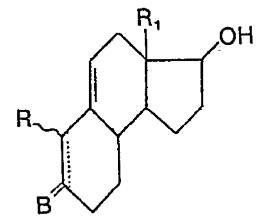

($III_B$)

in which B represents, either a ketal group, or an enamine group, or an enol ether, the dotted line represents a second bond when B is an enamine group or enol ether, which product is treated with an oxidizing agent so as to obtain a product with the formula ($IV_B$):

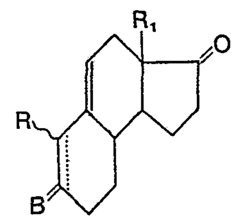

($IV_B$)

which is treated in the presence of a strong base, with a product with the formula $R'_4$ I so as to obtain a product with the formula ($V_B$)

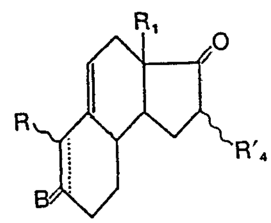

($V_B$)

which is submitted to the action of a reducing agent so as to obtain a product with the formula ($V'_B$):

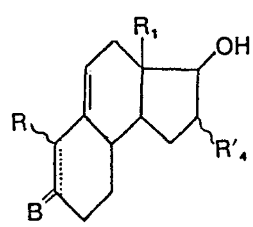

($V'_B$)

which is treated so as to eliminate the protector radical of the ketone function so as to obtain the sought product with the formula ($I'_B$);

3) To prepare the products with the formula ($I'_C$)

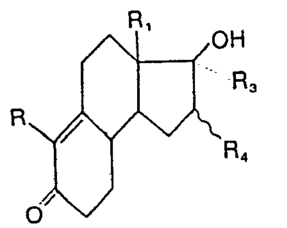

($I'_C$)

in which R, $R_1$, $R_3$ and $R_4$ have the previously mentioned significance it being understood that when $R_4$ represents a hydrogen atom, R and $R_1$ each can not represent a methyl radical, when $R_3$ represents an ethyl radical, a derivative with the formula $R_3$–D in which D represents a group Mg–Hal, in which Hal represents a halogen atom, or a lithium atom, is made to react with a derivative with the formula ($II_C$)

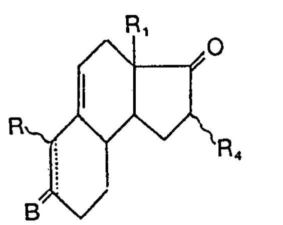

($II_C$)

in which B, R, $R_1$, $R_4$ and the dotted line have the previous significance and the product obtained is treated so as to eliminate the protector radical of the ketone function;

4) To prepare the products with the formula ($I'_D$)

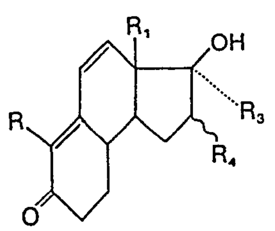

($I'_D$)

in which R, $R_1$, $R_3$ and $R_4$ have the previously mentioned significance, a reactive derivative of a protector group of the hydroxyl group is made to react with a product with the formula ($II_D$)

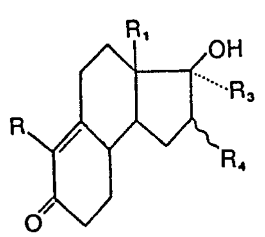

($II_D$)

in which R, $R_1$, $R_3$ and $R_4$ have the previously mentioned significance, so as to obtain a product with the formula ($III_D$)

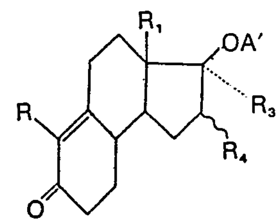

($III_D$)

in which A′ represents a protector radical of the hydroxyl group, which is treated with a bromiding reagent so as to obtain a product with the formula ($IV_D$):

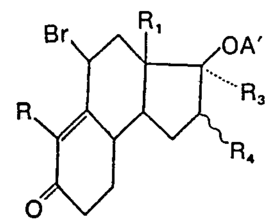

($IV_D$)

which is treated first with a debromhydration agent then with an agent which allows the protector group of the hydroxyl radical to be eliminated, so as to obtain the sought product $I'_D$;

5) To prepare the products with the formula ($I'_E$)

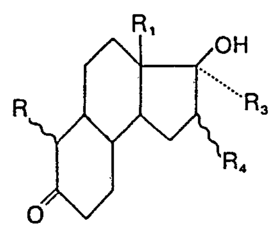

($I'_E$)

in which R, $R_1$, $R_3$ and $R_4$ have the previously mentioned significance, it being understood that when $R_4$ represents a hydrogen atom,

a) when $R_3$ represents a hydrogen atom, a methyl, ethyl, propargyl or isobutenyl radical, R and $R_1$ each can not represent a methyl radical,

b) when $R_3$ represents a hydrogen atom, R can not represent a methyl radical when $R_1$ represents an ethyl radical and conversely, R can not represent an ethyl radical when $R_1$ represents a methyl radical, a hydrogenation reagent is made to react with a product with the formula ($II'_E$):

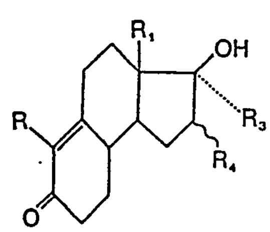

($II'_E$)

in which R, $R_1$, $R_3$ and $R_4$ have the previous significance;

6) To prepare the products with the formula ($I'_F$):

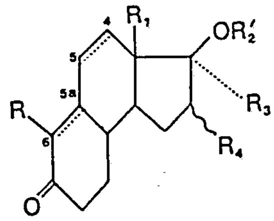

($I'_F$)

in which R, $R_1$, $R_3$, $R_4$ and the dotted lines have the previously mentioned significances and $R'_2$ has the significances of $R_2$ with the exception of the hydrogen value, it being understood that the products are excluded in which $R_4$ represents a hydrogen atom, the dotted line at position 4(5) does not indicate the presence of a second bond and the dotted line at position 5a(6) indicates the presence of a second bond and

a) R represents a methyl radical and $R_3$ represents a hydrogen atom and
   α) $R_1$ represents a methyl radical and $R_2$ represents an acetyl, benzoyl or tert-butyl radical,
   β) $R_1$ represents an ethyl radical and $R_2$ represents a tert-butyl radical,
b) R represents an ethyl or propyl radical, $R_1$ represents a methyl radical, $R_2$ represents a benzoyl radical and $R_3$ represents a hydrogen atom,

that the products are also excluded in which $R_4$ represents a hydrogen atom, the dotted lines do not indicate the presence of second bonds and

a) R and $R_1$ each represent a methyl radical, $R_3$ represents a hydrogen atom and $R'_2$ represents an acetyl or benzoyl radical,
b) $R_3$ represents a hydrogen atom, R and $R_1$ each represent a methyl radical and $R'_2$ represents a radical methoxymethyl,
c) R, $R_1$ and $R_3$ each represent a methyl radical and $R'_2$ represents an acetyl radical,

either a functional derivative with the formula $R'_2$–E in which E represents the residue of a functional group chosen from among the alkyl, alkenyl or alkynyl halides, or the halides or mixed or symmetric anhydrides of acyls and $R'_2$ has the previous significance, is made to react with a product with the formula ($II_F$):

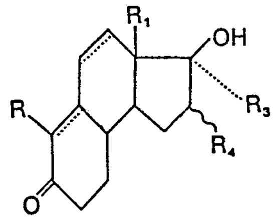

($II_F$)

in which R, $R_1$, $R_3$ and $R_4$ have the previous significance, it being understood that the values of the substituents R, $R_1$, $R'_2$, $R_3$, $R_4$ as well as the dotted lines are submitted to the previous restrictions, so as to obtain a product with the formula ($I'_F$) sought, or to prepare the products with the formula ($I'_F$) in which the dotted line at position 4(5) does not represent a second bond, a product with the formula ($II'_F$)

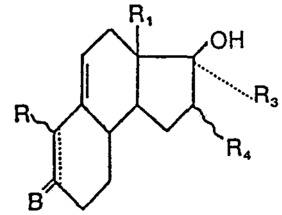

($II'_F$)

in which B has the previous significance and R, $R_1$, $R_3$ and $R_4$ are submitted to the previous restrictions, is made to react with a functional derivative with the formula

$$R'_2\text{–}E$$

in which $R'_2$ and E have the previous significance, so as to obtain a product with the formula ($III'_F$):

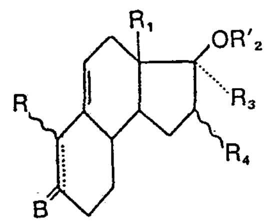

($III'_F$)

which is treated so as to eliminate the protector radical of the ketone function, so as to obtain the product with the formula ($I'_F$) in which the dotted line at position 4(5) does not represent a second bond.

**Patentansprüche für die Vertragsstaaten: BE, CH, DE, FR, GB, IT, LI, LU, NL, SE**

1. Als Arzneimittel und insbesondere als antiandrogene Arzneimittel die Produkte der allgemeinen Formel

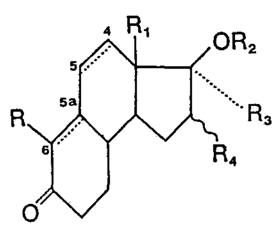

(I)

worin R einen linearen oder verzweigten Alkylrest mit 1 bis 4 Kohlenstoffatomen bedeutet, $R_1$ einen Methyl- oder Ethylrest darstellt, $R_2$ ein Wasserstoffatom, einen linearen oder verzweigten Alkyl-, Alkenyl- oder Alkinylrest mit höchstens 8 Kohlenstoffatomen, der gegebenenfalls durch ein Heteroatom unterbrochen ist, oder einen Formylrest oder Acylrest mit 2 bis 18 Kohlenstoffatomen, ausgewählt unter den Resten einer gesättigten oder ungesättigten, aliphatischen oder cycloaliphatischen Säure, den Cycloalkylalkansäureresten,

den Benzoyl- oder Phenylalkanoylresten, den linearen Acylresten mit einem Heteroatom, den 3-Pyridinylcarbonyl-, 4-Pyridinylcarbonyl-, Thiazolylcarbonyl-, 4,5-Dihydrothiazolylcarbonyl-, Oxazolylcarbonyl- oder Imidazolylcarbonylrest, bedeutet, $R_3$ ein Wasserstoffatom oder einen Alkyl-, Alkenyl- oder Alkinylrest mit höchstens 4 Kohlenstoffatomen bedeutet, $R_4$ ein Wasserstoffatom oder einen Alkylrest mit 1 bis 4 Kohlenstoffatomen bedeutet, wobei die gestrichelten Linien die etwaige Anwesenheit einer zweiten Bindung zwischen den 4(5)- und 5a(6)-Kohlenstoffatomen anzeigt, und die gewellte Linie anzeigt, dass sich der Substituent $R_4$ in der einen oder anderen der beiden möglichen α- oder β-Positionen befinden kann.

2. Als Arzneimittel die Produkte der Formel (I) gemäss Anspruch 1, worin R einen Methyl- oder Ethylrest bedeutet und $R_3$ und $R_4$ jeweils ein Wasserstoffatom bedeuten.

3. Als Arzneimittel die Produkte der Formel (I) gemäss einem der Ansprüche 1 oder 2, worin die gestrichelte Linie in 4(5)-Stellung nicht die Anwesenheit einer zweiten Bindung anzeigt und die gestrichelte Linie in 5a(6)-Stellung die Anwesenheit einer zweiten Bindung anzeigt.

4. Als Arzneimittel die Produkte der Formel (I) gemäss einem der Ansprüche 1 bis 3, worin $R_2$ ein Wasserstoffatom oder einen Methoxymethyl-, Allyl-, Acetyl- oder Butyrylrest bedeutet.

5. Als Arzneimittel:
das 3aβ-Methyl-3β-hydroxy-6-ethyl-1,2,3,3a,4,5,8,9,9a,9b-decahydro-7H-benz-(e)-inden-7-on,
das 3aβ-6-Diethyl-3β-hydroxy-1,2,3,3a,4,5,8,9,9a,9b-decahydro-7H-benz-(e)-inden-7-on,
das 3β-(Pyridin-3-yl)-carbonyloxy-6-ethyl-3aβ-methyl-1,2,3,3a,4,5,8,9,9a,9b-decahydro-7H-benz-(e)-inden-7-on.

6. Pharmazeutische Zusammensetzungen, enthaltend als Wirkstoff zumindest eines der Arzneimittel gemäss einem der Ansprüche 1 bis 5.

7. Die Produkte der allgemeinen Formel (I')

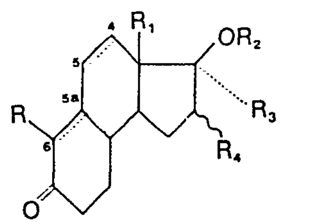

(I')

worin R, $R_1$, $R_2$, $R_3$, $R_z$ und die gestrichelten und gewellten Linien die in Anspruch 1 angegebenen Bedeutungen besitzen, mit Ausnahme der Produkte, worin $R_4$ ein Wasserstoffatom bedeutet, die gestrichelte Linie in 4(5)-Stellung nicht die Anwesenheit einer zweiten Bindung anzeigt, die gestrichelte Linie in 5a(6)-Stellung die Anwesenheit einer zweiten Bindung anzeigt und

a) R einen Methylrest bedeutet und $R_3$ ein Wasserstoffatom bedeutet und
  α) $R_2$ ein Wasserstoffatom, einen Acetyl-, Benzoyl- oder tert.-Butylrest bedeutet und $R_1$ einen Methylrest bedeutet,
  β) $R_2$ ein Wasserstoffatom oder einen tert.-Butylrest bedeutet und $R_1$ einen Ethylrest bedeutet,
b) R einen Ethyl- oder Propylrest bedeutet, $R_3$ ein Wasserstoffatom bedeutet, $R_2$ ein Wasserstoffatom oder einen Benzoylrest bedeutet und $R_1$ einen Methylrest bedeutet,
c) R einen Butylrest bedeutet, $R_3$ ein Wasserstoffatom bedeutet, $R_2$ ein Wasserstoffatom darstellt und $R_1$ einen Methylrest bedeutet,
d) R einen Methylrest bedeutet, $R_1$ einen Methylrest darstellt, $R_2$ ein Wasserstoffatom bedeutet und $R_3$ einen Ethyl- oder Methylrest bedeutet,

sowie mit Ausnahme der Produkte, worin $R_4$ ein Wasserstoffatom bedeutet und die gestrichelten Linien nicht die Anwesenheit zweiter Bindungen anzeigen und

a) R und $R_1$ jeweils einen Methylrest bedeuten, $R_3$ ein Wasserstoffatom darstellt und $R_2$ ein Wasserstoffatom oder einen Acetyl- oder Benzoylrest bedeutet,
b) R und $R_1$ jeweils einen Methylrest bedeuten, $R_2$ ein Wasserstoffatom bedeutet und $R_3$ einen Ethyl-, Propargyl- oder Isobutenylrest bedeutet,
c) $R_3$ ein Wasserstoffatom bedeutet und
  α) R und $R_1$ jeweils einen Methylrest bedeuten und $R_2$ einen Methoxymethylrest bedeutet,
  β) R einen Ethylrest bedeutet, $R_1$ einen Methylrest bedeutet und $R_2$ ein Wasserstoffatom darstellt,
  γ) R einen Methylrest bedeutet, $R_1$ einen Ethylrest bedeutet und $R_2$ ein Wasserstoffatom bedeutet,
d) R und $R_1$ jeweils einen Methylrest bedeuten, $R_2$ ein Wasserstoffatom oder einen Acetylrest bedeutet und $R_3$ einen Methylrest darstellt.

8. Die Produkte der allgemeinen Formel (I') gemäss Anspruch 7, worin $R_4$ einen Alkylrest mit 1 bis 4 Kohlenstoffatomen bedeutet.

9. Die Produkte der Formel (I') gemäss Anspruch 7, worin die gestrichelten Linien die Anwesenheit von zwei zweiten Bindungen in 4(5)- und 5a(6)-Stellung anzeigen.

10. Die Produkte der Formel (I') gemäss Anspruch 7, worin die gestrichelte Linie in 5a(6)-Stellung die Anwesenheit einer zweiten Bindung anzeigt und die Reste R und $R_1$ jeweils einen Ethylrest bedeuten.

11. 3aβ,6-Diethyl-3β-hydroxy-1,2,3,3a,4,5,8,9,9a,9b-decahydro-7H-benz-(e)-inden-7-on;

3β-(Pyridin-3-yl)-carbonyloxy-6-ethyl-3aβ-methyl-1,2,3,3a,4,5,8,9,9a,9b-decahydro-7H-benz-(e)-inden-7-on.

12. Verfahren zur Herstellung der Produkte der Formel (I') gemäss Anspruch 7, dadurch gekennzeichnet, dass man

1) zur Herstellung der Produkte der Formel ($I'_A$)

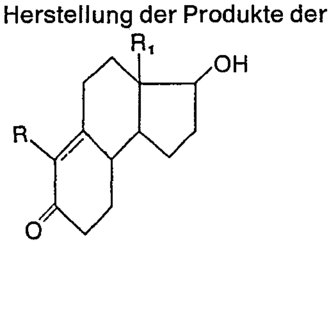

($I'_A$)

worin R und $R_1$ die in Anspruch 1 angegebene Bedeutung besitzen, wobei R nicht einen Methyl-, Ethyl-, n-Propyl- und n-Butylrest bedeuten kann, wenn $R_1$ einen Methylrest bedeutet, und R nicht einen Methylrest bedeuten kann, wenn $R_1$ einen Ethylrest bedeutet, ein Produkt der Formel ($II_A$)

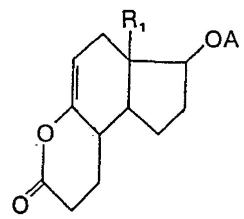

($II_A$)

mit einem Produkt der Formel ($III_A$)

$R{-}CH_2MgX$ ($III_A$)

worin R und $R_1$ die vorstehenden Bedeutungen besitzen, A einen Acylrest mit 2 bis 18 Kohlenstoffatomen bedeutet und X ein Halogenatom bedeutet, umsetzt und gewünschtenfalls das erhaltene Produkt verseift;

2) zur Herstellung der Produkte der Formel ($I'_B$)

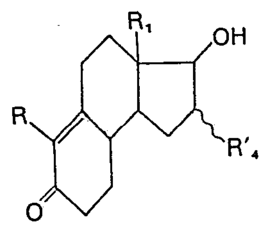

($I'_B$)

worin R und $R_1$ die in Anspruch 1 angegebene Bedeutung besitzen und $R'_4$ einen Alkylrest mit 1 bis 4 Kohlenstoffatomen darstellt, ein Produkt der Formel ($II_B$)

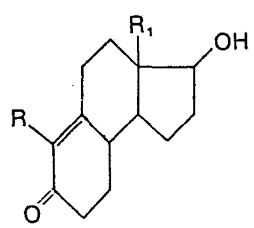

($II_B$)

worin R und $R_1$ die in Anspruch 1 angegebenen Bedeutungen besitzen, mit einem Reagens für den Schutz der Ketongruppe umsetzt, um ein Produkt der Formel ($III_B$)

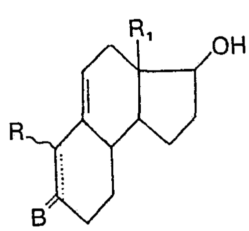

($III_B$)

zu erhalten, worin B entweder eine Ketalgruppe oder eine Enamingruppe oder einen Enolether bedeutet, wobei die gestrichelte Linie eine zweite Bindung anzeigt, wenn B eine Enamingruppe oder eine Enolethergruppe ist, welches Produkt man mit einem Oxidationsmittel behandelt, um ein Produkt der Formel ($IV_B$)

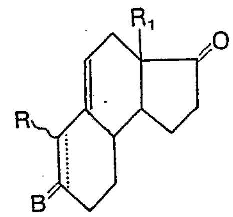

($IV_B$)

zu erhalten, das man in Gegenwart einer starken Base mit einem Produkt der Formel $R'_4J$ behandelt, um ein Produkt der Formel ($V_B$)

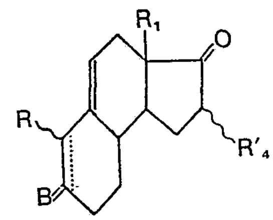

($V_B$)

zu erhalten, das man der Einwirkung eines Reduktionsmittels unterzieht, um ein Produkt der Formel ($V'_B$)

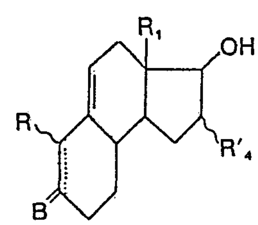

($V'_B$)

zu erhalten, das man derart behandelt, dass die Schutzgruppe für die Ketonfunktion eliminiert wird, um das gewünschte Produkt der Formel ($I'_B$) zu erhalten;

3) zur Herstellung der Produkte der Formel ($I'_C$)

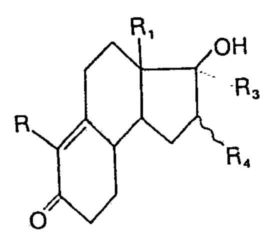

($I'_C$)

worin R, $R_1$, $R_3$ und $R_4$ die in Anspruch 1 angegebene Bedeutung besitzen, wobei, wenn $R_4$ ein Wasserstoffatom bedeutet, R und $R_1$ nicht jeweils einen Methylrest bedeuten können, wenn $R_3$ einen Ethylrest bedeutet, ein Derivat der Formel $R_3{-}D$, worin D eine Gruppe Mg-Hal, in der Hal ein Halogenatom darstellt, oder ein Lithiumatom bedeutet, mit einem Derivat der Formel ($II_C$)

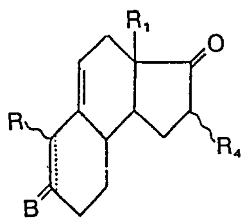

($II_C$)

worin B, R, $R_1$, $R_4$ und die gestrichelte Linie die angegebene Bedeutung besitzen, umsetzt, und das erhaltene Produkt so behandelt, dass die Schutzgruppe für die Ketonfunktion eliminiert wird;

4) zur Herstellung der Produkte der Formel ($I'_D$)

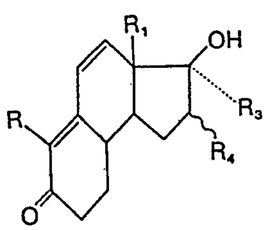

($I'_D$)

worin R, $R_1$, $R_3$ und $R_4$ die in Anspruch 1 angegebene Bedeutung besitzen, ein reaktives Derivat einer Schutzgruppe für die Hydroxylgruppe mit einem Produkt der Formel ($II_D$)

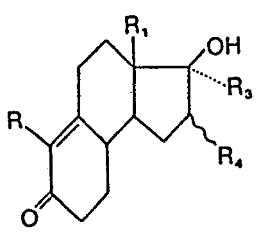

($II_D$)

worin R, $R_1$, $R_3$ und $R_4$ die in Anspruch 1 angegebene Bedeutung besitzen, umsetzt, um ein Produkt der Formel ($III_D$)

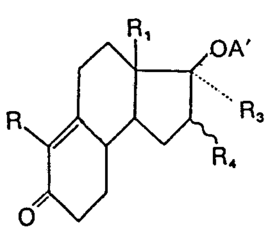

($III_D$)

zu erhalten, worin A' eine Schutzgruppe für die Hydroxylgruppe bedeutet, welches man mit einem Bromierungsreagens behandelt, um ein Produkt der Formel ($IV_D$)

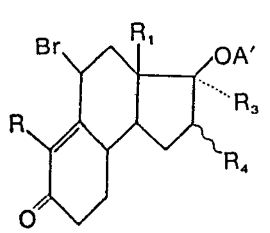

($IV_D$)

zu erhalten, das man zunächst mit einem Bromwasserstoff-Abspaltungsmittel und dann mit einem Mittel, das die Eliminierung der Schutzgruppe des Hydroxylrestes ermöglicht, behandelt, um das gewünschte Produkt $I'_D$ zu erhalten;

5) zur Herstellung der Produkte der Formel ($I'_E$)

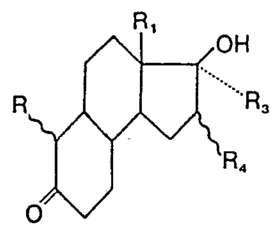

($I'_E$)

worin R, $R_1$, $R_3$ und $R_4$ die in Anspruch 1 angegebene Bedeutung besitzen, wobei, wenn $R_4$ ein Wasserstoffatom bedeutet,

a) wenn $R_3$ ein Wasserstoffatom, einen Methyl-, Ethyl-, Propargyl- oder Isobutenylrest bedeutet, R und $R_1$ nicht jeweils einen Methylrest bedeuten können,

b) wenn $R_3$ ein Wasserstoffatom bedeutet, R nicht einen Methylrest bedeuten kann, wenn $R_1$ einen Ethylrest bedeutet, und umgekehrt, R nicht einen Ethylrest bedeuten kann, wenn $R_1$ einen Methylrest bedeutet, ein Hydrierungsreagens mit einem Produkt der Formel ($II'_E$)

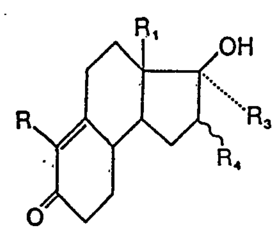

($II'_E$)

worin R, $R_1$, $R_3$ und $R_4$ die vorstehende Bedeutung besitzen, umsetzt;

6) zur Herstellung der Produkte der Formel ($I'_F$)

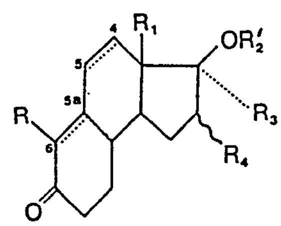

($I'_F$)

worin R, $R_1$, $R_4$ und die gestrichelten Linien die in Anspruch 1, angegebenen Bedeutungen besitzen und $R'_2$ die Bedeutungen von $R_2$ mit Ausnahme der Bedeutung Wasserstoff besitzt, wobei die Produkte ausgenommen sind, worin $R_4$ ein Wasserstoffatom bedeutet, die gestrichelte Linie in 4(5)-Stellung nicht die Anwesenheit einer zweiten Bindung anzeigt und die gestrichelte Linie in 5a(6)-Stellung die Anwesenheit einer zweiten Bindung anzeigt, und

a) R einen Methylrest bedeutet und $R_3$ ein Wasserstoffatom darstellt und

α) $R_1$ einen Methylrest bedeutet und $R'_2$ einen Acetyl-, Benzoyl- oder tert.-Butylrest bedeutet,

β) $R_1$ einen Ethylrest bedeutet und $R'_2$ einen tert.-Butylrest darstellt,

b) R einen Ethyl- oder Propylrest bedeutet, $R_1$ einen Methylrest bedeutet, $R'_2$ einen Benzoyl-

rest darstellt und $R_3$ ein Wasserstoffatom bedeutet,

und wobei auch die Produkte ausgenommen sind, worin $R_4$ ein Wasserstoffatom bedeutet, die gestrichelten Linien nicht die Anwesenheit von zweiten Bindungen anzeigen und

a) R und $R_1$ jeweils einen Methylrest bedeuten, $R_3$ ein Wasserstoffatom bedeutet und $R'_2$ einen Acetyl- oder Benzoylrest darstellt,

b) $R_3$ ein Wasserstoffatom bedeutet, R und $R_1$ jeweils einen Methylrest bedeuten und R'2 einen Methoxymethylrest bedeutet,

c) R, $R_1$ und $R_3$ jeweils einen Methylrest bedeuten und $R'_2$ einen Acetylrest darstellt,

entweder ein funktionelles Derivat der Formel $R'_2$–E, worin E den Rest einer funktionellen Gruppe, ausgewählt unter den Alkyl-, Alkenyl- oder Alkinylhalogeniden oder den Acylhalogeniden oder gemischten oder symmetrischen -anhydriden, bedeutet und $R'_2$ die vorstehende Bedeutung besitzt, mit einem Produkt der Formel ($II_F$)

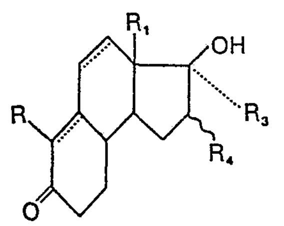

($II_F$)

worin R, $R_1$ und $R_4$ die vorstehende Bedeutung besitzen, wobei die Bedeutungen der Substituenten R, $R_1$, $R'_2$, $R_3$, $R_4$ sowie der gestrichelten Linien den vorstehenden Einschränkungen unterworfen sind, umsetzt, um das gewünschte Produkt der Formel ($I'_F$) zu erhalten,

oder zur Herstellung der Produkte der Formel ($I'_F$), worin die gestrichelte Linie in 4(5)-Stellung nicht die Anwesenheit einer zweiten Bindung anzeigt, ein Produkt der Formel ($II'_F$)

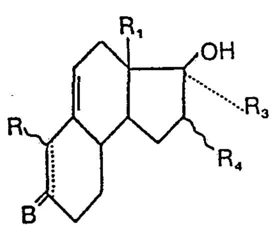

($II'_F$)

worin B die vorstehende Bedeutung besitzt und R, $R_1$, $R_3$ und $R_4$ den vorstehenden Einschränkungen unterzogen sind, mit einem funktionellen Derivat der Formel

$R'_2$–E

worin $R'_2$ und E die vorstehende Bedeutung besitzen, umsetzt, um ein Produkt der Formel ($III'_F$)

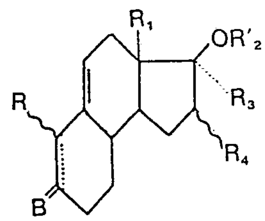

($III'_F$)

zu erhalten, das man derart behandelt, dass die Schutzgruppe für die Ketonfunktion eliminiert wird, um das Produkt der Formel ($I'_F$) zu erhalten, worin die gestrichelte Linie in 4(5)-Stellung keine zweite Bindung anzeigt.

13. Als Arzneimittel das 3aβ-Methyl-3β-acetoxy-6-ethyl-1,2,3,3a,4,5,8,9,9a,9b-decahydro-7H-benz-(e)-inden-7-on.

14. Pharmazeutische Zusammensetzungen, enthaltend als Wirkstoff das Arzneimittel gemäss Anspruch 13.

15. 3aβ-Methyl-3β-acetoxy-6-ethyl-1,2,3,3a,4,5,8,9,9a,9b-decahydro-7H-benz-(e)-inden-7-on.

**Patentanspruch für den Vertragsstaat: AT**

1. Verfahren zur Herstellung der Produkte der allgemeinen Formel (I')

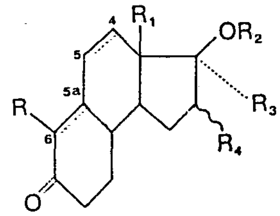

(I')

worin R einen linearen oder verzeigten Alkylrest mit 1 bis 4 Kohlenstoffatomen bedeutet, $R_1$ einen Methyl- oder Ethylrest bedeutet, $R_2$ ein Wasserstoffatom, einen linearen oder verzweigten Alkyl-, Alkenyl- oder Alkinylrest mit höchstens 8 Kohlenstoffatomen, der gegebenenfalls durch ein Heteroatom unterbrochen ist, oder einen Formylrest oder einen Acylrest mit 2 bis 28 Kohlenstoffatomen, ausgewählt unter den Resten einer gesättigten oder ungesättigten, aliphatischen oder cycloaliphatischen Säure, den Cycloalkylalkansäureresten, den Benzoyl- oder Phenylalkanoylresten, den linearen Acylresten mit einem Heteroatom, dem 3-Pyridinylcarbonyl-, 4-Pyridinylcarbonyl-, Thiazolylcarbonyl-, 4,5-Dihydrothiazolylcarbonyl-, Oxazolylcarbonyl- oder Imidazolylcarbonylrest, bedeutet, $R_3$ ein Wasserstoffatom oder einen Alkyl-, Alkenyl- oder Alkinylrest mit höchstens 4 Kohlenstoffatomen bedeutet, $R_4$ ein Wasserstoffatom oder einen Alyklrest mit 1 bis 4 Kohlenstoffatomen bedeutet, die gestrichelten Linien die etwaige Anwesenheit einer zweiten Bindung zwischen den 4(5)- und 5a(6)-Kohlenstoffatomen anzeigen, wobei die gewellte Linie anzeigt, dass der Substituent $R_4$ sich in der einen oder anderen der beiden möglichen α- oder β-Positionen befinden kann, mit Ausnahme der Produkte, worin $R_4$ ein

Wasserstoffatom bedeutet, die gestrichelte Linie in 4(5)-Stellung nicht die Anwesenheit einer zweiten Bindung anzeigt, die gestrichelte Linie in 5a(6)-Stellung die Anwesenheit einer zweiten Bindung anzeigt und

a) R einen Methylrest bedeutet und $R_3$ ein Wasserstoffatom bedeutet und
   α) $R_2$ ein Wasserstoffatom, einen Acetyl-, Benzoyl- oder tert.-Butylrest bedeutet und $R_1$ einen Methylrest darstellt,
   β) $R_2$ ein Wasserstoffatom oder einen tert.-Butylrest bedeutet und $R_1$ einen Ethylrest bedeutet,
b) R einen Ethyl- oder Propylrest bedeutet, $R_3$ Wasserstoffatom bedeutet, $R_2$ ein Wasserstoffatom oder einen Benzoylrest bedeutet und $R_1$ einen Methylrest darstellt,
c) R einen Butylrest bedeutet, $R_3$ ein Wasserstoffatom bedeutet, $R_2$ ein Wasserstoffatom darstellt und $R_1$ einen Methylrest bedeutet,
d) R einen Methylrest bedeutet, $R_1$ einen Methyl rest darstellt, $R_2$ ein Wasserstoffatom darstellt und $R_3$ einen Ethylrest bedeutet,

sowie mit Ausnahme der Produkte, worin $R_4$ ein Wasserstoffatom bedeutet und die gestrichelten Linien nicht die Anwesenheit von zweiten Bindungen anzeigen, und

a) R und $R_1$ jeweils einen Methylrest bedeuten, $R_3$ ein Wasserstoffatom bedeutet und $R_2$ ein Wasserstoffatom oder einen Acetyl- oder Benzoylrest bedeutet,
b) R und $R_1$ jeweils einen Methylrest bedeuten, $R_2$ ein Wasserstoffatom bedeutet und $R_3$ einen Ethyl-, Propargyl- oder Isobutenylrest bedeutet,
c) $R_3$ ein Wasserstoffatom bedeutet und
   α) R und $R_1$ jeweils einen Methylrest bedeuten und $R_2$ einen Methoxymethylrest bedeutet,
   β) R einen Ethylrest bedeutet, $R_1$ einen Methylrest bedeutet und $R_2$ ein Wasserstoffatom bedeutet,
   γ) R einen Methylrest bedeutet, $R_1$ einen Ethylrest darstellt und $R_2$ ein Wasserstoffatom bedeutet,
d) R und $R_1$ jeweils einen Methylrest bedeuten, $R_2$ ein Wasserstoffatom oder einen Acetylrest bedeutet und $R_3$ einen Methylrest bedeutet,

dadurch gekennzeichnet, dass

1) zur Herstellung der Produkte der Formel ($I'_A$)

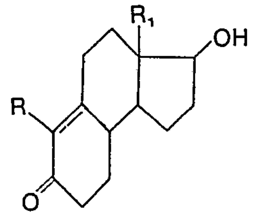

($I'_A$)

worin R und $R_1$ die vorstehend angegebene Bedeutung besitzen, wobei R nicht einen Methyl-, Ethyl-, n-Propyl- und n-Butylrest bedeuten kann, wenn $R_1$ einen Methylrest bedeutet, und R nicht einen Methylrest bedeuten kann, wenn $R_1$ einen Ethylrest bedeutet, ein Produkt der Formel ($II_A$)

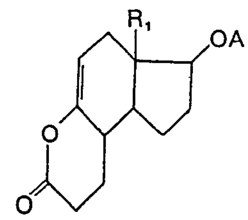

($II_A$)

mit einem Produkt der Formel ($III_A$)

$$R{-}CH_2MgX \qquad (III_A)$$

worin R und $R_1$ die vorstehenden Bedeutungen besitzen, A einen Acylrest mit 2 bis 18 Kohlenstoffatomen bedeutet und X ein Halogenatom darstellt, umsetzt und gewünschtenfalls das erhaltene Produkt verseift;

2) zur Herstellung der Produkte der Formel ($I'_B$)

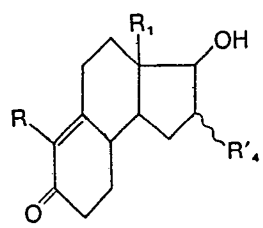

($I'_B$)

worin R und $R_1$ die vorstehend angegebene Bedeutung besitzen und $R'_4$ einen Alkylrest mit 1 bis 4 Kohlenstoffatomen bedeutet, ein Produkt der Formel ($II_B$)

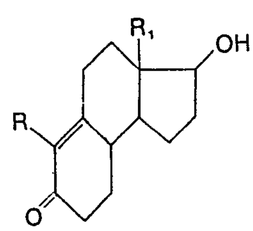

($II_B$)

worin R und $R_1$ die vorstehend angegebenen Bedeutungen besitzen, mit einem Reagens zum Schutz der Ketongruppe umsetzt, um ein Produkt der Formel ($III_B$)

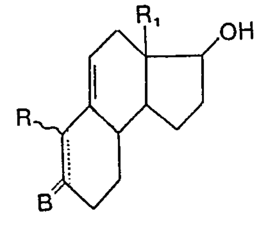

($III_B$)

zu erhalten, worin B entweder eine Ketalgruppe oder eine Enamingruppe oder eine Enolethergruppe bedeutet, wobei die gestrichelte Linie eine zweite Bindung anzeigt, wenn B eine Enamingruppe oder eine Enolethergruppe darstellt, welches Produkt man mit einem Oxidationsmittel behandelt, um ein Produkt der Formel ($IV_B$)

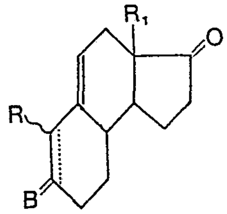

$(IV_B)$

zu erhalten, das man in Gegenwart einer starken Base mit einem Produkt der Formel $R'_4J$ behandelt, um ein Produkt der Formel $(V_B)$

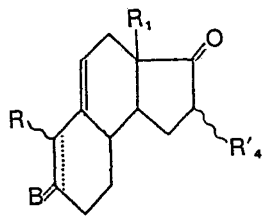

$(V_B)$

zu erhalten, das man der Einwirkung eines Reduktionsmittels unterzieht, um ein Produkt der Formel $(V'_B)$

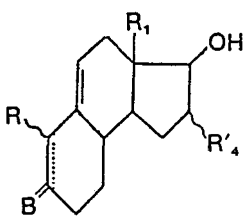

$(V'_B)$

zu erhalten, das man derart behandelt, dass die Schutzgruppe für die Ketonfunktion eliminiert wird, um das gewünschte Produkt der Formel $(I'_B)$ zu erhalten;

3) zur Herstellung der Produkte der Formel $(I'_C)$

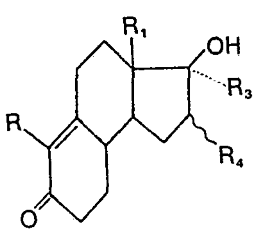

$(I'_C)$

worin R, $R_1$, $R_z$ und $R_4$ die vorstehend angegebene Bedeutung besitzen, wobei, wenn $R_4$ ein Wasserstoffatom bedeutet, R und $R_1$ nicht jeweils einen Methylrest bedeuten können, wenn $R_3$ einen Ethylrest bedeutet, ein Derivat der Formel $R_3$–D, worin D eine Gruppe Mg–Hal, worin Hal ein Halogenatom darstellt, oder ein Lithiumatom bedeutet, mit einem Derivat der Formel $(II_C)$

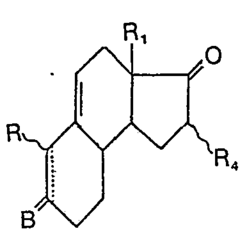

$(II_C)$

worin B, R, $R_1$, $R_4$ und die gestrichelte Linie die vorstehende Bedeutung besitzen, umsetzt und das erhaltene Produkt derart behandelt, dass die Schutzgruppe für die Ketonfunktion eliminiert wird;

4) zur Herstellung der Produkte der Formel $(I'_D)$

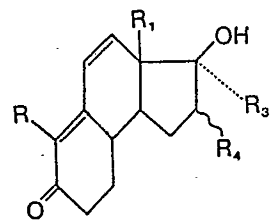

$(I'_D)$

worin R, $R_1$, $R_3$ und $R_4$ die vorstehende Bedeutung besitzen, ein reaktives Derivat einer Schutzgruppe für die Hydroxylgruppe mit einem Produkt der Formel $(II_D)$

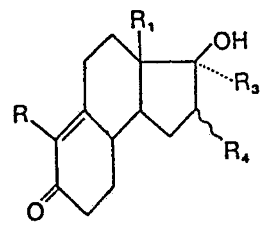

$(II_D)$

worin R, $R_1$, $R_3$ und $R_4$ die vorstehende Bedeutung besitzen, umsetzt, um ein Produkt der Formel $(III_D)$

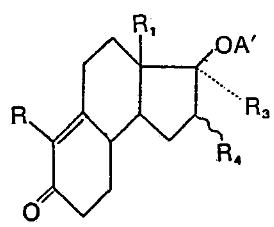

$(III_D)$

worin A' eine Schutzgruppe für die Hydroxylgruppe bedeutet, zu erhalten, welches man mit einem Bromierungsreagens behandelt, um ein Produkt der Formel $(IV_D)$

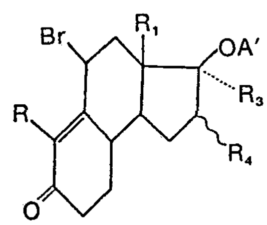

$(IV_D)$

zu erhalten, das man zunächst mit einem Bromwasserstoff-Abspaltungsmittel und danach mit einem Mittel, das es ermöglicht, die Schutzgruppe für die Hydroxylgruppe zu eliminieren, behandelt, um das gewünschte Produkt $I'_D$ zu erhalten;

5) zur Herstellung der Produkte der Formel $(I'_E)$

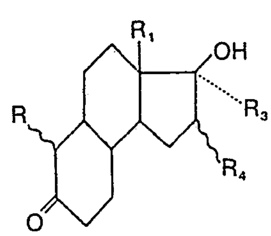

$(I'_E)$

worin R, $R_1$, $R_3$ und $R_4$ die vorstehend angegebene Bedeutung besitzen, wobei, wenn $R_4$ ein Wasserstoffatom bedeutet,

a) wenn $R_3$ ein Wasserstoffatom, einen Methyl-, Ethyl-, Propargyl- oder Isobutenylrest bedeutet, R und $R_1$ nicht jeweils einen Methylrest bedeuten können,
b) wenn $R_3$ ein Wasserstoffatom bedeutet, R nicht einen Methylrest bedeuten kann, wenn $R_1$ einen Ethylrest darstellt, und umgekehrt, R nicht einen Ethylrest bedeuten kann, wenn $R_1$ einen Methylrest darstellt,

ein Hydrierungsreagens mit einem Produkt der Formel ($II'_E$)

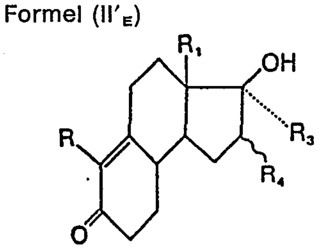

($II'_E$)

worin R, $R_1$, $R_3$ und $R_4$ die vorstehende Bedeutung besitzen, umsetzt;

6) zur Herstellung der Produkte der Formel ($I'_F$)

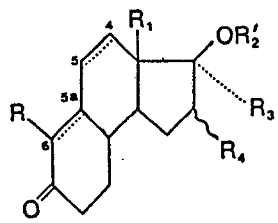

($I'_F$)

worin R, $R_1$, $R_3$, $R_4$ und die gestrichelten Linien die vorstehend angegebenen Bedeutungen besitzen und $R'_2$ die Bedeutungen von $R_2$ mit Ausnahme der Bedeutung Wasserstoff besitzt, wobei die Produkte ausgenommen sind, worin $R_4$ ein Wasserstoffatom darstellt, die gestrichelte Linie in 4(5)-Stellung nicht die Anwesenheit einer zweiten Bindung anzeigt und die gestrichelte Linie in 5a(6)-Stellung die Anwesenheit einer zweiten Bindung anzeigt und

a) R einen Methylrest bedeutet und $R_3$ eine Wasserstoffatom darstellt und
   α) $R_1$ einen Methylrest bedeutet und $R_2$ einen Acetyl-, Benzoyl- oder tert.-Butylrest bedeutet,
   β) $R_1$ einen Ethylrest bedeutet und $R_2$ einen tert.-Butylrest bedeutet,
b) R einen Ethyl- oder Propylrest bedeutet, $R_1$ einen Methylrest bedeutet, $R_2$ einen Benzoylrest darstellt und $R_3$ ein Wasserstoffatom bedeutet,

sowie mit Ausnahme der Produkte, worin $R_4$ ein Wasserstoffatom bedeutet, die gestrichelten Linien nicht die Anwesenheit einer zweiten Bindung anzeigen und

a) R und $R_1$ jeweils einen Methylrest bedeuten, $R_3$ ein Wasserstoffatom darstellt und $R'_2$ einen Acetyl- oder Benzoylrest bedeutet,
b) $R_3$ ein Wasserstoffatom bedeutet, R und $R_1$ jeweils einen Methylrest bedeuten und $R'_2$ einen Methoxymethylrest darstellt,
c) R, $R_1$ und $R_3$ jeweils einen Methylrest bedeuten und $R'_2$ einen Acetylrest bedeutet,

entweder ein funktionelles Derivat der Formel $R'_2$–E, worin E den Rest einer funktionellen Gruppe, ausgewählt unter den Alkyl-, Alkenyl- oder Alkinylhalogeniden oder den Acylhalogeniden oder gemischten oder symmetrischen -anhydriden, darstellt und $R'_2$ die vorstehende Bedeutung besitzt, mit einem Produkt der Formel ($II_F$)

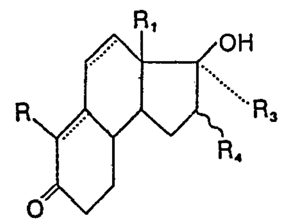

($II_F$)

worin R, $R_1$, $R_3$ und $R_4$ die vorstehende Bedeutung besitzen, wobei die Bedeutungen der Substituenten R, $R_1$, $R'_2$, $R_3$, $R_4$ sowie der gestrichelten Linien den vorstehenden Einschränkungen unterzogen sind, umsetzt, um das gewünschte Produkt der Formel ($I'_F$) zu erhalten;

oder zur Herstellung der Produkte der Formel ($I'_F$), worin die gestrichelte Linie in 4(5)-Stellung nicht eine zweite Bindung anzeigt, ein Produkt der Formel ($II'_F$)

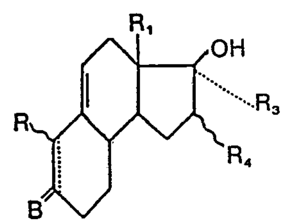

($II'_F$)

worin B die vorstehende Bedeutung besitzt und R, $R_1$, $R_3$ und $R_4$ den vorstehenden Einschränkungen unterzogen sind, mit einem funktionellen Derivat der Formel

$R'_2$–E

worin $R'_2$ und E die vorstehende Bedeutung besitzen, umsetzt, um ein Produkt der Formel ($III'_F$)

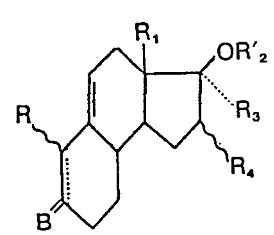

($III'_F$)

zu erhalten, das man derart behandelt, dass die Schutzgruppe für die Ketonfunktion eliminiert wird, um das Produkt der Formel ($I'_F$) zu erhalten, worin die gestrichelte Linie in 4(5)-Stellung nicht eine zweite Bindung anzeigt.